# EUROPEAN PATENT APPLICATION

(11) **EP 2 180 035 A1**
(43) Date of publication of application: **28.04.2010**
(21) Application number: 09178946.1
(22) Date of filing: 12.07.2001
(51) Int. Cl.: C11D 3/386, C12N 9/28

(54) **Alpha-amylase mutants with altered properties**

(30) Priority: 01.08.2000 DK 200001160; 12.09.2000 DK 200001354; 10.11.2000 DK 200001687; 26.04.2001 DK 200100655
(62) Divisional of application: 01956424.4
(71) Applicant: Novozymes A/S, 2880 Bagsvaerd (DK)
(72) Inventor: Thisted, Thomas, 2960 Rungsted Kyst (DK); Kjaerulf, Soeren, 2720 Vanloese (DK); Fuglsang, Claus Crone, 2990 Nivaa (DK)

(57) **Abstract**

The present invention relates to variants (mutants) of parent Termamyl-like alpha-amylases, which variant has alpha-amylase activity and exhibits altered stability, in particular at high temperatures and/or at low pH relative, and/or low Ca2+ to the parent alpha-amylase.

SEQUENCE LISTING
<110> Novo Nordisk A/S

<120>

<130>

<160> 28

<170> PatentIn Ver. 2.1

<210> 1
<211> 1455
<212> DNA
<213> Bacillus sp.

<220>
<221> CDS
<222> (1)..(1455)
<223> SP690

<400> 1
cat cat aat gga aca aat ggt act atg atg caa tat ttc gaa tgg tat    48
His His Asn Gly Thr Asn Gly Thr Met Met Gln Tyr Phe Glu Trp Tyr
  1               5                  10                  15

ttg cca aat gac ggg aat cat tgg aac agg ttg agg gat gac gca gct    96
Leu Pro Asn Asp Gly Asn His Trp Asn Arg Leu Arg Asp Asp Ala Ala
             20                  25                  30

aac tta aag agt aaa ggg ata aca gct gta tgg atc cca cct gca tgg    144
Asn Leu Lys Ser Lys Gly Ile Thr Ala Val Trp Ile Pro Pro Ala Trp
         35                  40                  45

aag ggg act tcc cag aat gat gta ggt tat gga gcc tat gat tta tat    192
Lys Gly Thr Ser Gln Asn Asp Val Gly Tyr Gly Ala Tyr Asp Leu Tyr
     50                  55                  60

gat ctt gga gag ttt aac cag aag ggg acg gtt cgt aca aaa tat gga    240
Asp Leu Gly Glu Phe Asn Gln Lys Gly Thr Val Arg Thr Lys Tyr Gly
 65                  70                  75                  80

aca cgc aac cag cta cag gct gcg gtg acc tct tta aaa aat aac ggc    288
Thr Arg Asn Gln Leu Gln Ala Ala Val Thr Ser Leu Lys Asn Asn Gly
                 85                  90                  95

att cag gta tat ggt gat gtc gtc atg aat cat aaa ggt gga gca gat    336
Ile Gln Val Tyr Gly Asp Val Val Met Asn His Lys Gly Gly Ala Asp
            100                 105                 110

ggt acg gaa att gta aat gcg gta gaa gtg aat cgg agc aac cga aac    384
Gly Thr Glu Ile Val Asn Ala Val Glu Val Asn Arg Ser Asn Arg Asn
        115                 120                 125

cag gaa acc tca gga gag tat gca ata gaa gcg tgg aca aag ttt gat    432
Gln Glu Thr Ser Gly Glu Tyr Ala Ile Glu Ala Trp Thr Lys Phe Asp
    130                 135                 140

ttt cct gga aga gga aat aac cat tcc agc ttt aag tgg cgc tgg tat    480
Phe Pro Gly Arg Gly Asn Asn His Ser Ser Phe Lys Trp Arg Trp Tyr
145                 150                 155                 160

cat ttt gat ggg aca gat tgg gat cag tca cgc cag ctt caa aac aaa    528
His Phe Asp Gly Thr Asp Trp Asp Gln Ser Arg Gln Leu Gln Asn Lys
                165                 170                 175

ata tat aaa ttc agg gga aca ggc aag gcc tgg gac tgg gaa gtc gat    576
Ile Tyr Lys Phe Arg Gly Thr Gly Lys Ala Trp Asp Trp Glu Val Asp

180                 185                 190

aca gag aat ggc aac tat gac tat ctt atg tat gca gac gtg gat atg     624
Thr Glu Asn Gly Asn Tyr Asp Tyr Leu Met Tyr Ala Asp Val Asp Met
        195                 200                 205

gat cac cca gaa gta ata cat gaa ctt aga aac tgg gga gtg tgg tat     672
Asp His Pro Glu Val Ile His Glu Leu Arg Asn Trp Gly Val Trp Tyr
    210                 215                 220

acg aat aca ctg aac ctt gat gga ttt aga ata gat gca gtg aaa cat     720
Thr Asn Thr Leu Asn Leu Asp Gly Phe Arg Ile Asp Ala Val Lys His
225                 230                 235                 240

ata aaa tat agc ttt acg aga gat tgg ctt aca cat gtg cgt aac acc     768
Ile Lys Tyr Ser Phe Thr Arg Asp Trp Leu Thr His Val Arg Asn Thr
                245                 250                 255

aca ggt aaa cca atg ttt gca gtg gct gag ttt tgg aaa aat gac ctt     816
Thr Gly Lys Pro Met Phe Ala Val Ala Glu Phe Trp Lys Asn Asp Leu
            260                 265                 270

ggt gca att gaa aac tat ttg aat aaa aca agt tgg aat cac tcg gtg     864
Gly Ala Ile Glu Asn Tyr Leu Asn Lys Thr Ser Trp Asn His Ser Val
        275                 280                 285

ttt gat gtt cct ctc cac tat aat ttg tac aat gca tct aat agc ggt     912
Phe Asp Val Pro Leu His Tyr Asn Leu Tyr Asn Ala Ser Asn Ser Gly
    290                 295                 300

ggt tat tat gat atg aga aat att tta aat ggt tct gtg gtg caa aaa     960
Gly Tyr Tyr Asp Met Arg Asn Ile Leu Asn Gly Ser Val Val Gln Lys
305                 310                 315                 320

cat cca aca cat gcc gtt act ttt gtt gat aac cat gat tct cag ccc     1008
His Pro Thr His Ala Val Thr Phe Val Asp Asn His Asp Ser Gln Pro
                325                 330                 335

ggg gaa gca ttg gaa tcc ttt gtt caa caa tgg ttt aaa cca ctt gca     1056
Gly Glu Ala Leu Glu Ser Phe Val Gln Gln Trp Phe Lys Pro Leu Ala
            340                 345                 350

tat gca ttg gtt ctg aca agg gaa caa ggt tat cct tcc gta ttt tat     1104
Tyr Ala Leu Val Leu Thr Arg Glu Gln Gly Tyr Pro Ser Val Phe Tyr
        355                 360                 365

ggg gat tac tac ggt atc cca acc cat ggt gtt ccg gct atg aaa tct     1152
Gly Asp Tyr Tyr Gly Ile Pro Thr His Gly Val Pro Ala Met Lys Ser
    370                 375                 380

aaa ata gac cct ctt ctg cag gca cgt caa act ttt gcc tat ggt acg     1200
Lys Ile Asp Pro Leu Leu Gln Ala Arg Gln Thr Phe Ala Tyr Gly Thr
385                 390                 395                 400

cag cat gat tac ttt gat cat cat gat att atc ggt tgg aca aga gag     1248
Gln His Asp Tyr Phe Asp His His Asp Ile Ile Gly Trp Thr Arg Glu
                405                 410                 415

gga aat agc tcc cat cca aat tca ggc ctt gcc acc att atg tca gat     1296
Gly Asn Ser Ser His Pro Asn Ser Gly Leu Ala Thr Ile Met Ser Asp
            420                 425                 430

ggt cca ggt ggt aac aaa tgg atg tat gtg ggg aaa aat aaa gcg gga     1344
Gly Pro Gly Gly Asn Lys Trp Met Tyr Val Gly Lys Asn Lys Ala Gly
        435                 440                 445

caa gtt tgg aga gat att acc gga aat agg aca ggc acc gtc aca att     1392
Gln Val Trp Arg Asp Ile Thr Gly Asn Arg Thr Gly Thr Val Thr Ile
    450                 455                 460

aat gca gac gga tgg ggt aat ttc tct gtt aat gga ggg tcc gtt tcg     1440
Asn Ala Asp Gly Trp Gly Asn Phe Ser Val Asn Gly Gly Ser Val Ser
465                 470                 475                 480

gtt tgg gtg aag caa                                                 1455
Val Trp Val Lys Gln
                485

<210> 2
<211> 485
<212> PRT
<213> Bacillus sp.

<400> 2
His His Asn Gly Thr Asn Gly Thr Met Met Gln Tyr Phe Glu Trp Tyr
1               5                   10                  15

Leu Pro Asn Asp Gly Asn His Trp Asn Arg Leu Arg Asp Asp Ala Ala
            20                  25                  30

Asn Leu Lys Ser Lys Gly Ile Thr Ala Val Trp Ile Pro Pro Ala Trp
        35                  40                  45

Lys Gly Thr Ser Gln Asn Asp Val Gly Tyr Gly Ala Tyr Asp Leu Tyr
    50                  55                  60

Asp Leu Gly Glu Phe Asn Gln Lys Gly Thr Val Arg Thr Lys Tyr Gly
65                  70                  75                  80

Thr Arg Asn Gln Leu Gln Ala Ala Val Thr Ser Leu Lys Asn Asn Gly
                85                  90                  95

Ile Gln Val Tyr Gly Asp Val Val Met Asn His Lys Gly Gly Ala Asp
            100                 105                 110

Gly Thr Glu Ile Val Asn Ala Val Glu Val Asn Arg Ser Asn Arg Asn
        115                 120                 125

Gln Glu Thr Ser Gly Glu Tyr Ala Ile Glu Ala Trp Thr Lys Phe Asp
    130                 135                 140

Phe Pro Gly Arg Gly Asn Asn His Ser Ser Phe Lys Trp Arg Trp Tyr
145                 150                 155                 160

His Phe Asp Gly Thr Asp Trp Asp Gln Ser Arg Gln Leu Gln Asn Lys
                165                 170                 175

Ile Tyr Lys Phe Arg Gly Thr Gly Lys Ala Trp Asp Trp Glu Val Asp
            180                 185                 190

Thr Glu Asn Gly Asn Tyr Asp Tyr Leu Met Tyr Ala Asp Val Asp Met
        195                 200                 205

Asp His Pro Glu Val Ile His Glu Leu Arg Asn Trp Gly Val Trp Tyr
    210                 215                 220

Thr Asn Thr Leu Asn Leu Asp Gly Phe Arg Ile Asp Ala Val Lys His
225                 230                 235                 240

Ile Lys Tyr Ser Phe Thr Arg Asp Trp Leu Thr His Val Arg Asn Thr
                245                 250                 255

Thr Gly Lys Pro Met Phe Ala Val Ala Glu Phe Trp Lys Asn Asp Leu
            260                 265                 270

Gly Ala Ile Glu Asn Tyr Leu Asn Lys Thr Ser Trp Asn His Ser Val
        275                 280                 285

Phe Asp Val Pro Leu His Tyr Asn Leu Tyr Asn Ala Ser Asn Ser Gly
    290                 295                 300

Gly Tyr Tyr Asp Met Arg Asn Ile Leu Asn Gly Ser Val Val Gln Lys
305                 310                 315                 320

His Pro Thr His Ala Val Thr Phe Val Asp Asn His Asp Ser Gln Pro
                325                 330                 335

Gly Glu Ala Leu Glu Ser Phe Val Gln Gln Trp Phe Lys Pro Leu Ala
            340                 345                 350

Tyr Ala Leu Val Leu Thr Arg Glu Gln Gly Tyr Pro Ser Val Phe Tyr
        355                 360                 365

Gly Asp Tyr Tyr Gly Ile Pro Thr His Gly Val Pro Ala Met Lys Ser
    370                 375                 380

Lys Ile Asp Pro Leu Leu Gln Ala Arg Gln Thr Phe Ala Tyr Gly Thr
385                 390                 395                 400

Gln His Asp Tyr Phe Asp His His Asp Ile Ile Gly Trp Thr Arg Glu
                405                 410                 415

Gly Asn Ser Ser His Pro Asn Ser Gly Leu Ala Thr Ile Met Ser Asp
            420                 425                 430

Gly Pro Gly Gly Asn Lys Trp Met Tyr Val Gly Lys Asn Lys Ala Gly
        435                 440                 445

Gln Val Trp Arg Asp Ile Thr Gly Asn Arg Thr Gly Thr Val Thr Ile
    450                 455                 460

Asn Ala Asp Gly Trp Gly Asn Phe Ser Val Asn Gly Gly Ser Val Ser
465                 470                 475                 480

Val Trp Val Lys Gln
                485



<210> 3
<211> 1455
<212> DNA
<213> Bacillus sp.

<220>
<221> CDS
<222> (1)..(1455)
<223> SP722

<400> 3
cat cat aat ggg aca aat ggg acg atg atg caa tac ttt gaa tgg cac      48
His His Asn Gly Thr Asn Gly Thr Met Met Gln Tyr Phe Glu Trp His
1               5                   10                  15

ttg cct aat gat ggg aat cac tgg aat aga tta aga gat gat gct agt      96
Leu Pro Asn Asp Gly Asn His Trp Asn Arg Leu Arg Asp Asp Ala Ser
            20                  25                  30

aat cta aga aat aga ggt ata acc gct att tgg att ccg cct gcc tgg      144
Asn Leu Arg Asn Arg Gly Ile Thr Ala Ile Trp Ile Pro Pro Ala Trp
        35                  40                  45

aaa ggg act tcg caa aat gat gtg ggg tat gga gcc tat gat ctt tat      192
Lys Gly Thr Ser Gln Asn Asp Val Gly Tyr Gly Ala Tyr Asp Leu Tyr
    50                  55                  60

gat tta ggg gaa ttt aat caa aag ggg acg gtt cgt act aag tat ggg     240
Asp Leu Gly Glu Phe Asn Gln Lys Gly Thr Val Arg Thr Lys Tyr Gly
 65                  70                  75                  80

aca cgt agt caa ttg gag tct gcc atc cat gct tta aag aat aat ggc     288
Thr Arg Ser Gln Leu Glu Ser Ala Ile His Ala Leu Lys Asn Asn Gly
                 85                  90                  95

gtt caa gtt tat ggg gat gta gtg atg aac cat aaa gga gga gct gat     336
Val Gln Val Tyr Gly Asp Val Val Met Asn His Lys Gly Gly Ala Asp
            100                 105                 110

gct aca gaa aac gtt ctt gct gtc gag gtg aat cca aat aac cgg aat     384
Ala Thr Glu Asn Val Leu Ala Val Glu Val Asn Pro Asn Asn Arg Asn
        115                 120                 125

caa gaa ata tct ggg gac tac aca att gag gct tgg act aag ttt gat     432
Gln Glu Ile Ser Gly Asp Tyr Thr Ile Glu Ala Trp Thr Lys Phe Asp
    130                 135                 140

ttt cca ggg agg ggt aat aca tac tca gac ttt aaa tgg cgt tgg tat     480
Phe Pro Gly Arg Gly Asn Thr Tyr Ser Asp Phe Lys Trp Arg Trp Tyr
145                 150                 155                 160

cat ttc gat ggt gta gat tgg gat caa tca cga caa ttc caa aat cgt     528
His Phe Asp Gly Val Asp Trp Asp Gln Ser Arg Gln Phe Gln Asn Arg
                165                 170                 175

atc tac aaa ttc cga ggt gat ggt aag gca tgg gat tgg gaa gta gat     576
Ile Tyr Lys Phe Arg Gly Asp Gly Lys Ala Trp Asp Trp Glu Val Asp
            180                 185                 190

tcg gaa aat gga aat tat gat tat tta atg tat gca gat gta gat atg     624
Ser Glu Asn Gly Asn Tyr Asp Tyr Leu Met Tyr Ala Asp Val Asp Met
        195                 200                 205

gat cat ccg gag gta gta aat gag ctt aga aga tgg gga gaa tgg tat     672
Asp His Pro Glu Val Val Asn Glu Leu Arg Arg Trp Gly Glu Trp Tyr
    210                 215                 220

aca aat aca tta aat ctt gat gga ttt agg atc gat gcg gtg aag cat     720
Thr Asn Thr Leu Asn Leu Asp Gly Phe Arg Ile Asp Ala Val Lys His
225                 230                 235                 240

att aaa tat agc ttt aca cgt gat tgg ttg acc cat gta aga aac gca     768
Ile Lys Tyr Ser Phe Thr Arg Asp Trp Leu Thr His Val Arg Asn Ala
                245                 250                 255

acg gga aaa gaa atg ttt gct gtt gct gaa ttt tgg aaa aat gat tta     816
Thr Gly Lys Glu Met Phe Ala Val Ala Glu Phe Trp Lys Asn Asp Leu
            260                 265                 270

ggt gcc ttg gag aac tat tta aat aaa aca aac tgg aat cat tct gtc     864
Gly Ala Leu Glu Asn Tyr Leu Asn Lys Thr Asn Trp Asn His Ser Val
        275                 280                 285

ttt gat gtc ccc ctt cat tat aat ctt tat aac gcg tca aat agt gga     912
Phe Asp Val Pro Leu His Tyr Asn Leu Tyr Asn Ala Ser Asn Ser Gly
    290                 295                 300

ggc aac tat gac atg gca aaa ctt ctt aat gga acg gtt gtt caa aag     960
Gly Asn Tyr Asp Met Ala Lys Leu Leu Asn Gly Thr Val Val Gln Lys
305                 310                 315                 320

cat cca atg cat gcc gta act ttt gtg gat aat cac gat tct caa cct     1008
His Pro Met His Ala Val Thr Phe Val Asp Asn His Asp Ser Gln Pro
                325                 330                 335

ggg gaa tca tta gaa tca ttt gta caa gaa tgg ttt aag cca ctt gct     1056

Gly Glu Ser Leu Glu Ser Phe Val Gln Glu Trp Phe Lys Pro Leu Ala
            340                 345                 350

tat gcg ctt att tta aca aga gaa caa ggc tat ccc tct gtc ttc tat      1104
Tyr Ala Leu Ile Leu Thr Arg Glu Gln Gly Tyr Pro Ser Val Phe Tyr
        355                 360                 365

ggt gac tac tat gga att cca aca cat agt gtc cca gca atg aaa gcc      1152
Gly Asp Tyr Tyr Gly Ile Pro Thr His Ser Val Pro Ala Met Lys Ala
    370                 375                 380

aag att gat cca atc tta gag gcg cgt caa aat ttt gca tat gga aca      1200
Lys Ile Asp Pro Ile Leu Glu Ala Arg Gln Asn Phe Ala Tyr Gly Thr
385                 390                 395                 400

caa cat gat tat ttt gac cat cat aat ata atc gga tgg aca cgt gaa      1248
Gln His Asp Tyr Phe Asp His His Asn Ile Ile Gly Trp Thr Arg Glu
                405                 410                 415

gga aat acc acg cat ccc aat tca gga ctt gcg act atc atg tcg gat      1296
Gly Asn Thr Thr His Pro Asn Ser Gly Leu Ala Thr Ile Met Ser Asp
            420                 425                 430

ggg cca ggg gga gag aaa tgg atg tac gta ggg caa aat aaa gca ggt      1344
Gly Pro Gly Gly Glu Lys Trp Met Tyr Val Gly Gln Asn Lys Ala Gly
        435                 440                 445

caa gtt tgg cat gac ata act gga aat aaa cca gga aca gtt acg atc      1392
Gln Val Trp His Asp Ile Thr Gly Asn Lys Pro Gly Thr Val Thr Ile
    450                 455                 460

aat gca gat gga tgg gct aat ttt tca gta aat gga gga tct gtt tcc      1440
Asn Ala Asp Gly Trp Ala Asn Phe Ser Val Asn Gly Gly Ser Val Ser
465                 470                 475                 480

att tgg gtg aaa cga                                                  1455
Ile Trp Val Lys Arg
                485


<210> 4
<211> 485
<212> PRT
<213> Bacillus sp.

<400> 4
His His Asn Gly Thr Asn Gly Thr Met Met Gln Tyr Phe Glu Trp His
1               5                   10                  15

Leu Pro Asn Asp Gly Asn His Trp Asn Arg Leu Arg Asp Asp Ala Ser
            20                  25                  30

Asn Leu Arg Asn Arg Gly Ile Thr Ala Ile Trp Ile Pro Pro Ala Trp
        35                  40                  45

Lys Gly Thr Ser Gln Asn Asp Val Gly Tyr Gly Ala Tyr Asp Leu Tyr
    50                  55                  60

Asp Leu Gly Glu Phe Asn Gln Lys Gly Thr Val Arg Thr Lys Tyr Gly
65                  70                  75                  80

Thr Arg Ser Gln Leu Glu Ser Ala Ile His Ala Leu Lys Asn Asn Gly
                85                  90                  95

Val Gln Val Tyr Gly Asp Val Val Met Asn His Lys Gly Gly Ala Asp
            100                 105                 110

Ala Thr Glu Asn Val Leu Ala Val Glu Val Asn Pro Asn Asn Arg Asn
        115                 120                 125

Gln Glu Ile Ser Gly Asp Tyr Thr Ile Glu Ala Trp Thr Lys Phe Asp
    130                 135                 140

Phe Pro Gly Arg Gly Asn Thr Tyr Ser Asp Phe Lys Trp Arg Trp Tyr
145                 150                 155                 160

His Phe Asp Gly Val Asp Trp Asp Gln Ser Arg Gln Phe Gln Asn Arg
                165                 170                 175

Ile Tyr Lys Phe Arg Gly Asp Gly Lys Ala Trp Asp Trp Glu Val Asp
            180                 185                 190

Ser Glu Asn Gly Asn Tyr Asp Tyr Leu Met Tyr Ala Asp Val Asp Met
        195                 200                 205

Asp His Pro Glu Val Val Asn Glu Leu Arg Arg Trp Gly Glu Trp Tyr
    210                 215                 220

Thr Asn Thr Leu Asn Leu Asp Gly Phe Arg Ile Asp Ala Val Lys His
225                 230                 235                 240

Ile Lys Tyr Ser Phe Thr Arg Asp Trp Leu Thr His Val Arg Asn Ala
                245                 250                 255

Thr Gly Lys Glu Met Phe Ala Val Ala Glu Phe Trp Lys Asn Asp Leu
            260                 265                 270

Gly Ala Leu Glu Asn Tyr Leu Asn Lys Thr Asn Trp Asn His Ser Val
        275                 280                 285

Phe Asp Val Pro Leu His Tyr Asn Leu Tyr Asn Ala Ser Asn Ser Gly
    290                 295                 300

Gly Asn Tyr Asp Met Ala Lys Leu Leu Asn Gly Thr Val Val Gln Lys
305                 310                 315                 320

His Pro Met His Ala Val Thr Phe Val Asp Asn His Asp Ser Gln Pro
                325                 330                 335

Gly Glu Ser Leu Glu Ser Phe Val Gln Glu Trp Phe Lys Pro Leu Ala
            340                 345                 350

Tyr Ala Leu Ile Leu Thr Arg Glu Gln Gly Tyr Pro Ser Val Phe Tyr
        355                 360                 365

Gly Asp Tyr Tyr Gly Ile Pro Thr His Ser Val Pro Ala Met Lys Ala
    370                 375                 380

Lys Ile Asp Pro Ile Leu Glu Ala Arg Gln Asn Phe Ala Tyr Gly Thr
385                 390                 395                 400

Gln His Asp Tyr Phe Asp His His Asn Ile Ile Gly Trp Thr Arg Glu
                405                 410                 415

Gly Asn Thr Thr His Pro Asn Ser Gly Leu Ala Thr Ile Met Ser Asp
            420                 425                 430

Gly Pro Gly Gly Glu Lys Trp Met Tyr Val Gly Gln Asn Lys Ala Gly
        435                 440                 445

Gln Val Trp His Asp Ile Thr Gly Asn Lys Pro Gly Thr Val Thr Ile
    450                 455                 460

Asn Ala Asp Gly Trp Ala Asn Phe Ser Val Asn Gly Gly Ser Val Ser
465                 470                 475                 480

Ile Trp Val Lys Arg
                485

<210> 5
<211> 1548
<212> DNA
<213> Bacillus stearothermophilus

<220>
<221> CDS
<222> (1)..(1548)
<223> BSG

<400> 5
gcc gca ccg ttt aac ggc acc atg atg cag tat ttt gaa tgg tac ttg     48
Ala Ala Pro Phe Asn Gly Thr Met Met Gln Tyr Phe Glu Trp Tyr Leu
1               5                   10                  15

ccg gat gat ggc acg tta tgg acc aaa gtg gcc aat gaa gcc aac aac     96
Pro Asp Asp Gly Thr Leu Trp Thr Lys Val Ala Asn Glu Ala Asn Asn
            20                  25                  30

tta tcc agc ctt ggc atc acc gct ctt tgg ctg ccg ccc gct tac aaa     144
Leu Ser Ser Leu Gly Ile Thr Ala Leu Trp Leu Pro Pro Ala Tyr Lys
        35                  40                  45

gga aca agc cgc agc gac gta ggg tac gga gta tac gac ttg tat gac     192
Gly Thr Ser Arg Ser Asp Val Gly Tyr Gly Val Tyr Asp Leu Tyr Asp
    50                  55                  60

ctc ggc gaa ttc aat caa aaa ggg acc gtc cgc aca aaa tac gga aca     240
Leu Gly Glu Phe Asn Gln Lys Gly Thr Val Arg Thr Lys Tyr Gly Thr
65                  70                  75                  80

aaa gct caa tat ctt caa gcc att caa gcc gcc cac gcc gct gga atg     288
Lys Ala Gln Tyr Leu Gln Ala Ile Gln Ala Ala His Ala Ala Gly Met
                85                  90                  95

caa gtg tac gcc gat gtc gtg ttc gac cat aaa ggc ggc gct gac ggc     336
Gln Val Tyr Ala Asp Val Val Phe Asp His Lys Gly Gly Ala Asp Gly
            100                 105                 110

acg gaa tgg gtg gac gcc gtc gaa gtc aat ccg tcc gac cgc aac caa     384
Thr Glu Trp Val Asp Ala Val Glu Val Asn Pro Ser Asp Arg Asn Gln
        115                 120                 125

gaa atc tcg ggc acc tat caa atc caa gca tgg acg aaa ttt gat ttt     432
Glu Ile Ser Gly Thr Tyr Gln Ile Gln Ala Trp Thr Lys Phe Asp Phe
    130                 135                 140

ccc ggg cgg ggc aac acc tac tcc agc ttt aag tgg cgc tgg tac cat     480
Pro Gly Arg Gly Asn Thr Tyr Ser Ser Phe Lys Trp Arg Trp Tyr His
145                 150                 155                 160

ttt gac ggc gtt gat tgg gac gaa agc cga aaa ttg agc cgc att tac     528
Phe Asp Gly Val Asp Trp Asp Glu Ser Arg Lys Leu Ser Arg Ile Tyr
                165                 170                 175

aaa ttc cgc ggc atc ggc aaa gcg tgg gat tgg gaa gta gac acg gaa     576
Lys Phe Arg Gly Ile Gly Lys Ala Trp Asp Trp Glu Val Asp Thr Glu
            180                 185                 190

aac gga aac tat gac tac tta atg tat gcc gac ctt gat atg gat cat     624
Asn Gly Asn Tyr Asp Tyr Leu Met Tyr Ala Asp Leu Asp Met Asp His
        195                 200                 205

ccc gaa gtc gtg acc gag ctg aaa aac tgg ggg aaa tgg tat gtc aac     672
Pro Glu Val Val Thr Glu Leu Lys Asn Trp Gly Lys Trp Tyr Val Asn
    210                 215                 220

aca acg aac att gat ggg ttc cgg ctt gat gcc gtc aag cat att aag 720
Thr Thr Asn Ile Asp Gly Phe Arg Leu Asp Ala Val Lys His Ile Lys
225                 230                 235                 240

ttc agt ttt ttt cct gat tgg ttg tcg tat gtg cgt tct cag act ggc 768
Phe Ser Phe Phe Pro Asp Trp Leu Ser Tyr Val Arg Ser Gln Thr Gly
                245                 250                 255

aag ccg cta ttt acc gtc ggg gaa tat tgg agc tat gac atc aac aag 816
Lys Pro Leu Phe Thr Val Gly Glu Tyr Trp Ser Tyr Asp Ile Asn Lys
            260                 265                 270

ttg cac aat tac att acg aaa aca gac gga acg atg tct ttg ttt gat 864
Leu His Asn Tyr Ile Thr Lys Thr Asp Gly Thr Met Ser Leu Phe Asp
        275                 280                 285

gcc ccg tta cac aac aaa ttt tat acc gct tcc aaa tca ggg ggc gca 912
Ala Pro Leu His Asn Lys Phe Tyr Thr Ala Ser Lys Ser Gly Gly Ala
    290                 295                 300

ttt gat atg cgc acg tta atg acc aat act ctc atg aaa gat caa ccg 960
Phe Asp Met Arg Thr Leu Met Thr Asn Thr Leu Met Lys Asp Gln Pro
305                 310                 315                 320

aca ttg gcc gtc acc ttc gtt gat aat cat gac acc gaa ccc ggc caa 1008
Thr Leu Ala Val Thr Phe Val Asp Asn His Asp Thr Glu Pro Gly Gln
                325                 330                 335

gcg ctg cag tca tgg gtc gac cca tgg ttc aaa ccg ttg gct tac gcc 1056
Ala Leu Gln Ser Trp Val Asp Pro Trp Phe Lys Pro Leu Ala Tyr Ala
            340                 345                 350

ttt att cta act cgg cag gaa gga tac ccg tgc gtc ttt tat ggt gac 1104
Phe Ile Leu Thr Arg Gln Glu Gly Tyr Pro Cys Val Phe Tyr Gly Asp
        355                 360                 365

tat tat ggc att cca caa tat aac att cct tcg ctg aaa agc aaa atc 1152
Tyr Tyr Gly Ile Pro Gln Tyr Asn Ile Pro Ser Leu Lys Ser Lys Ile
    370                 375                 380

gat ccg ctc ctc atc gcg cgc agg gat tat gct tac gga acg caa cat 1200
Asp Pro Leu Leu Ile Ala Arg Arg Asp Tyr Ala Tyr Gly Thr Gln His
385                 390                 395                 400

gat tat ctt gat cac tcc gac atc atc ggg tgg aca agg gaa ggg ggc 1248
Asp Tyr Leu Asp His Ser Asp Ile Ile Gly Trp Thr Arg Glu Gly Gly
                405                 410                 415

act gaa aaa cca gga tcc gga ctg gcc gca ctg atc acc gat ggg ccg 1296
Thr Glu Lys Pro Gly Ser Gly Leu Ala Ala Leu Ile Thr Asp Gly Pro
            420                 425                 430

gga gga agc aaa tgg atg tac gtt ggc aaa caa cac gct gga aaa gtg 1344
Gly Gly Ser Lys Trp Met Tyr Val Gly Lys Gln His Ala Gly Lys Val
        435                 440                 445

ttc tat gac ctt acc ggc aac cgg agt gac acc gtc acc atc aac agt 1392
Phe Tyr Asp Leu Thr Gly Asn Arg Ser Asp Thr Val Thr Ile Asn Ser
    450                 455                 460

gat gga tgg ggg gaa ttc aaa gtc aat ggc ggt tcg gtt tcg gtt tgg 1440
Asp Gly Trp Gly Glu Phe Lys Val Asn Gly Gly Ser Val Ser Val Trp
465                 470                 475                 480

gtt cct aga aaa acg acc gtt tct acc atc gct cgg ccg atc aca acc 1488
Val Pro Arg Lys Thr Thr Val Ser Thr Ile Ala Arg Pro Ile Thr Thr
                485                 490                 495

cga ccg tgg act ggt gaa ttc gtc cgt tgg acc gaa cca cgg ttg gtg     1536
Arg Pro Trp Thr Gly Glu Phe Val Arg Trp Thr Glu Pro Arg Leu Val
            500                 505                 510

gca tgg cct tga                                                     1548
Ala Trp Pro
        515


<210> 6
<211> 515
<212> PRT
<213> Bacillus stearothermophilus

<400> 6
Ala Ala Pro Phe Asn Gly Thr Met Met Gln Tyr Phe Glu Trp Tyr Leu
1               5                   10                  15

Pro Asp Asp Gly Thr Leu Trp Thr Lys Val Ala Asn Glu Ala Asn Asn
            20                  25                  30

Leu Ser Ser Leu Gly Ile Thr Ala Leu Trp Leu Pro Pro Ala Tyr Lys
        35                  40                  45

Gly Thr Ser Arg Ser Asp Val Gly Tyr Gly Val Tyr Asp Leu Tyr Asp
    50                  55                  60

Leu Gly Glu Phe Asn Gln Lys Gly Thr Val Arg Thr Lys Tyr Gly Thr
65                  70                  75                  80

Lys Ala Gln Tyr Leu Gln Ala Ile Gln Ala Ala His Ala Ala Gly Met
                85                  90                  95

Gln Val Tyr Ala Asp Val Val Phe Asp His Lys Gly Gly Ala Asp Gly
            100                 105                 110

Thr Glu Trp Val Asp Ala Val Glu Val Asn Pro Ser Asp Arg Asn Gln
        115                 120                 125

Glu Ile Ser Gly Thr Tyr Gln Ile Gln Ala Trp Thr Lys Phe Asp Phe
    130                 135                 140

Pro Gly Arg Gly Asn Thr Tyr Ser Ser Phe Lys Trp Arg Trp Tyr His
145                 150                 155                 160

Phe Asp Gly Val Asp Trp Asp Glu Ser Arg Lys Leu Ser Arg Ile Tyr
                165                 170                 175

Lys Phe Arg Gly Ile Gly Lys Ala Trp Asp Trp Glu Val Asp Thr Glu
            180                 185                 190

Asn Gly Asn Tyr Asp Tyr Leu Met Tyr Ala Asp Leu Asp Met Asp His
        195                 200                 205

Pro Glu Val Val Thr Glu Leu Lys Asn Trp Gly Lys Trp Tyr Val Asn
    210                 215                 220

Thr Thr Asn Ile Asp Gly Phe Arg Leu Asp Ala Val Lys His Ile Lys
225                 230                 235                 240

Phe Ser Phe Phe Pro Asp Trp Leu Ser Tyr Val Arg Ser Gln Thr Gly
                245                 250                 255

Lys Pro Leu Phe Thr Val Gly Glu Tyr Trp Ser Tyr Asp Ile Asn Lys
            260                 265                 270

Leu His Asn Tyr Ile Thr Lys Thr Asp Gly Thr Met Ser Leu Phe Asp
        275                 280                 285

Ala Pro Leu His Asn Lys Phe Tyr Thr Ala Ser Lys Ser Gly Gly Ala
    290                 295                 300

Phe Asp Met Arg Thr Leu Met Thr Asn Thr Leu Met Lys Asp Gln Pro
305                 310                 315                 320

Thr Leu Ala Val Thr Phe Val Asp Asn His Asp Thr Glu Pro Gly Gln
                325                 330                 335

Ala Leu Gln Ser Trp Val Asp Pro Trp Phe Lys Pro Leu Ala Tyr Ala
            340                 345                 350

Phe Ile Leu Thr Arg Gln Glu Gly Tyr Pro Cys Val Phe Tyr Gly Asp
        355                 360                 365

Tyr Tyr Gly Ile Pro Gln Tyr Asn Ile Pro Ser Leu Lys Ser Lys Ile
    370                 375                 380

Asp Pro Leu Leu Ile Ala Arg Arg Asp Tyr Ala Tyr Gly Thr Gln His
385                 390                 395                 400

Asp Tyr Leu Asp His Ser Asp Ile Ile Gly Trp Thr Arg Glu Gly Gly
                405                 410                 415

Thr Glu Lys Pro Gly Ser Gly Leu Ala Ala Leu Ile Thr Asp Gly Pro
            420                 425                 430

Gly Gly Ser Lys Trp Met Tyr Val Gly Lys Gln His Ala Gly Lys Val
        435                 440                 445

Phe Tyr Asp Leu Thr Gly Asn Arg Ser Asp Thr Val Thr Ile Asn Ser
    450                 455                 460

Asp Gly Trp Gly Glu Phe Lys Val Asn Gly Gly Ser Val Ser Val Trp
465                 470                 475                 480

Val Pro Arg Lys Thr Thr Val Ser Thr Ile Ala Arg Pro Ile Thr Thr
                485                 490                 495

Arg Pro Trp Thr Gly Glu Phe Val Arg Trp Thr Glu Pro Arg Leu Val
            500                 505                 510

Ala Trp Pro
        515

<210> 7
<211> 1920
<212> DNA
<213> Bacillus licheniformis

<220>
<221> CDS
<222> (421)..(1872)
<223> Termamyl

<400> 7
cggaagattg gaagtacaaa aataagcaaa agattgtcaa tcatgtcatg agccatgcgg 60
gagacggaaa aatcgtctta atgcacgata tttatgcaac gttcgcagat gctgctgaag 120
agattattaa aaagctgaaa gcaaaaggct atcaattggt aactgtatct cagcttgaag 180
aagtgaagaa gcagagaggc tattgaataa atgagtagaa gcgccatatc ggcgcttttc 240
ttttggaaga aaatataggg aaaatggtac ttgttaaaaa ttcggaatat ttatacaaca 300
tcatatgttt cacattgaaa ggggaggaga atcatgaaac aacaaaaacg gctttacgcc 360
cgattgctga cgctgttatt tgcgctcatc ttcttgctgc ctcattctgc agcagcggcg 420
gca aat ctt aat ggg acg ctg atg cag tat ttt gaa tgg tac atg ccc 468
aat gac ggc caa cat tgg agg cgt ttg caa aac gac tcg gca tat ttg 516
gct gaa cac ggt att act gcc gtc tgg att ccc ccg gca tat aag gga 564
acg agc caa gcg gat gtg ggc tac ggt gct tac gac ctt tat gat tta 612
ggg gag ttt cat caa aaa ggg acg gtt cgg aca aag tac ggc aca aaa 660
gga gag ctg caa tct gcg atc aaa agt ctt cat tcc cgc gac att aac 708
gtt tac ggg gat gtg gtc atc aac cac aaa ggc ggc gct gat gcg acc 756
gaa gat gta acc gcg gtt gaa gtc gat ccc gct gac cgc aac cgc gta 804
att tca gga gaa cac cta att aaa gcc tgg aca cat ttt cat ttt ccg 852
ggg cgc ggc agc aca tac agc gat ttt aaa tgg cat tgg tac cat ttt 900
gac gga acc gat tgg gac gag tcc cga aag ctg aac cgc atc tat aag 948
ttt caa gga aag gct tgg gat tgg gaa gtt tcc aat gaa aac ggc aac 996
tat gat tat ttg atg tat gcc gac atc gat tat gac cat cct gat gtc 1044
gca gca gaa att aag aga tgg ggc act tgg tat gcc aat gaa ctg caa 1092
ttg gac ggt ttc cgt ctt gat gct gtc aaa cac att aaa ttt tct ttt 1140
ttg cgg gat tgg gtt aat cat gtc agg gaa aaa acg ggg aag gaa atg 1188
ttt acg gta gct gaa tat tgg cag aat gac ttg ggc gcg ctg gaa aac 1236
tat ttg aac aaa aca aat ttt aat cat tca gtg ttt gac gtg ccg ctt 1284
cat tat cag ttc cat gct gca tcg aca cag gga ggc ggc tat gat atg 1332
agg aaa ttg ctg aac ggt acg gtc gtt tcc aag cat ccg ttg aaa tcg 1380
gtt aca ttt gtc gat aac cat gat aca cag ccg ggg caa tcg ctt gag 1428
tcg act gtc caa aca tgg ttt aag ccg ctt gct tac gct ttt att ctc 1476
aca agg gaa tct gga tac cct cag gtt ttc tac ggg gat atg tac ggg 1524
acg aaa gga gac tcc cag cgc gaa att cct gcc ttg aaa cac aaa att 1572
gaa ccg atc tta aaa gcg aga aaa cag tat gcg tac gga gca cag cat 1620
gat tat ttc gac cac cat gac att gtc ggc tgg aca agg gaa ggc gac 1668
agc tcg gtt gca aat tca ggt ttg gcg gca tta ata aca gac gga ccc 1716
ggt ggg gca aag cga atg tat gtc ggc cgg caa aac gcc ggt gag aca 1764
tgg cat gac att acc gga aac cgt tcg gag ccg gtt gtc atc aat tcg 1812
gaa ggc tgg gga gag ttt cac gta aac ggc ggg tcg gtt tca att tat 1860
gtt caa aga tag aagagcagag aggacggatt tcctgaagga aatccgtttt 1912
tttatttt 1920

<210> 8
<211> 483
<212> PRT
<213> Bacillus licheniformis

<400> 8

<210> 9
<211> 2084
<212> DNA
<213> Bacillus amyloliquefaciens

<220>
<221> CDS
<222> (343)..(1794)
<223> BAN

<400> 9
gccccgcaca tacgaaaaga ctggctgaaa acattgagcc tttgatgact gatgatttgg 60

ctgaagaagt ggatcgattg tttgagaaaa gaagaagacc ataaaaatac cttgtctgtc 120

atcagacagg gtatttttta tgctgtccag actgtccgct gtgtaaaaat aaggaataaa 180

ggggggttgt tattatttta ctgatatgta aaatataatt tgtataagaa aatgagaggg 240

agaggaaaca tgattcaaaa acgaaagcgg acagtttcgt tcagacttgt gcttatgtgc 300

acgctgttat ttgtcagttt gccgattaca aaaacatcag cc gta aat ggc acg    354
                                              Val Asn Gly Thr
                                              1

ctg atg cag tat ttt gaa tgg tat acg ccg aac gac ggc cag cat tgg    402
Leu Met Gln Tyr Phe Glu Trp Tyr Thr Pro Asn Asp Gly Gln His Trp
5                   10                  15                  20

aaa cga ttg cag aat gat gcg gaa cat tta tcg gat atc gga atc act    450
Lys Arg Leu Gln Asn Asp Ala Glu His Leu Ser Asp Ile Gly Ile Thr
                25                  30                  35

gcc gtc tgg att cct ccc gca tac aaa gga ttg agc caa tcc gat aac    498
Ala Val Trp Ile Pro Pro Ala Tyr Lys Gly Leu Ser Gln Ser Asp Asn
            40                  45                  50

gga tac gga cct tat gat ttg tat gat tta gga gaa ttc cag caa aaa    546
Gly Tyr Gly Pro Tyr Asp Leu Tyr Asp Leu Gly Glu Phe Gln Gln Lys
        55                  60                  65

ggg acg gtc aga acg aaa tac ggc aca aaa tca gag ctt caa gat gcg    594
Gly Thr Val Arg Thr Lys Tyr Gly Thr Lys Ser Glu Leu Gln Asp Ala
    70                  75                  80

atc ggc tca ctg cat tcc cgg aac gtc caa gta tac gga gat gtg gtt    642
Ile Gly Ser Leu His Ser Arg Asn Val Gln Val Tyr Gly Asp Val Val

85                  90                  95                  100

ttg aat cat aag gct ggt gct gat gca aca gaa gat gta act gcc gtc     690
Leu Asn His Lys Ala Gly Ala Asp Ala Thr Glu Asp Val Thr Ala Val
                105                 110                 115

gaa gtc aat ccg gcc aat aga aat cag gaa act tcg gag gaa tat caa     738
Glu Val Asn Pro Ala Asn Arg Asn Gln Glu Thr Ser Glu Glu Tyr Gln
            120                 125                 130

atc aaa gcg tgg acg gat ttt cgt ttt ccg ggc cgt gga aac acg tac     786
Ile Lys Ala Trp Thr Asp Phe Arg Phe Pro Gly Arg Gly Asn Thr Tyr
        135                 140                 145

agt gat ttt aaa tgg cat tgg tat cat ttc gac gga gcg gac tgg gat     834
Ser Asp Phe Lys Trp His Trp Tyr His Phe Asp Gly Ala Asp Trp Asp
    150                 155                 160

gaa tcc cgg aag atc agc cgc atc ttt aag ttt cgt ggg gaa gga aaa     882
Glu Ser Arg Lys Ile Ser Arg Ile Phe Lys Phe Arg Gly Glu Gly Lys
165                 170                 175                 180

gcg tgg gat tgg gaa gta tca agt gaa aac ggc aac tat gac tat tta     930
Ala Trp Asp Trp Glu Val Ser Ser Glu Asn Gly Asn Tyr Asp Tyr Leu
                185                 190                 195

atg tat gct gat gtt gac tac gac cac cct gat gtc gtg gca gag aca     978
Met Tyr Ala Asp Val Asp Tyr Asp His Pro Asp Val Val Ala Glu Thr
            200                 205                 210

aaa aaa tgg ggt atc tgg tat gcg aat gaa ctg tca tta gac ggc ttc     1026
Lys Lys Trp Gly Ile Trp Tyr Ala Asn Glu Leu Ser Leu Asp Gly Phe
        215                 220                 225

cgt att gat gcc gcc aaa cat att aaa ttt tca ttt ctg cgt gat tgg     1074
Arg Ile Asp Ala Ala Lys His Ile Lys Phe Ser Phe Leu Arg Asp Trp
    230                 235                 240

gtt cag gcg gtc aga cag gcg acg gga aaa gaa atg ttt acg gtt gcg     1122
Val Gln Ala Val Arg Gln Ala Thr Gly Lys Glu Met Phe Thr Val Ala
245                 250                 255                 260

gag tat tgg cag aat aat gcc ggg aaa ctc gaa aac tac ttg aat aaa     1170
Glu Tyr Trp Gln Asn Asn Ala Gly Lys Leu Glu Asn Tyr Leu Asn Lys
                265                 270                 275

aca agc ttt aat caa tcc gtg ttt gat gtt ccg ctt cat ttc aat tta     1218
Thr Ser Phe Asn Gln Ser Val Phe Asp Val Pro Leu His Phe Asn Leu
            280                 285                 290

cag gcg gct tcc tca caa gga ggc gga tat gat atg agg cgt ttg ctg     1266
Gln Ala Ala Ser Ser Gln Gly Gly Gly Tyr Asp Met Arg Arg Leu Leu
        295                 300                 305

gac ggt acc gtt gtg tcc agg cat ccg gaa aag gcg gtt aca ttt gtt     1314
Asp Gly Thr Val Val Ser Arg His Pro Glu Lys Ala Val Thr Phe Val
    310                 315                 320

gaa aat cat gac aca cag ccg gga cag tca ttg gaa tcg aca gtc caa     1362
Glu Asn His Asp Thr Gln Pro Gly Gln Ser Leu Glu Ser Thr Val Gln
325                 330                 335                 340

act tgg ttt aaa ccg ctt gca tac gcc ttt att ttg aca aga gaa tcc     1410
Thr Trp Phe Lys Pro Leu Ala Tyr Ala Phe Ile Leu Thr Arg Glu Ser
                345                 350                 355

ggt tat cct cag gtg ttc tat ggg gat atg tac ggg aca aaa ggg aca     1458
Gly Tyr Pro Gln Val Phe Tyr Gly Asp Met Tyr Gly Thr Lys Gly Thr
            360                 365                 370

tcg cca aag gaa att ccc tca ctg aaa gat aat ata gag ccg att tta    1506
Ser Pro Lys Glu Ile Pro Ser Leu Lys Asp Asn Ile Glu Pro Ile Leu
        375                 380                 385

aaa gcg cgt aag gag tac gca tac ggg ccc cag cac gat tat att gac    1554
Lys Ala Arg Lys Glu Tyr Ala Tyr Gly Pro Gln His Asp Tyr Ile Asp
    390                 395                 400

cac ccg gat gtg atc gga tgg acg agg gaa ggt gac agc tcc gcc gcc    1602
His Pro Asp Val Ile Gly Trp Thr Arg Glu Gly Asp Ser Ser Ala Ala
405                 410                 415                 420

aaa tca ggt ttg gcc gct tta atc acg gac gga ccc ggc gga tca aag    1650
Lys Ser Gly Leu Ala Ala Leu Ile Thr Asp Gly Pro Gly Gly Ser Lys
                425                 430                 435

cgg atg tat gcc ggc ctg aaa aat gcc ggc gag aca tgg tat gac ata    1698
Arg Met Tyr Ala Gly Leu Lys Asn Ala Gly Glu Thr Trp Tyr Asp Ile
            440                 445                 450

acg ggc aac cgt tca gat act gta aaa atc gga tct gac ggc tgg gga    1746
Thr Gly Asn Arg Ser Asp Thr Val Lys Ile Gly Ser Asp Gly Trp Gly
        455                 460                 465

gag ttt cat gta aac gat ggg tcc gtc tcc att tat gtt cag aaa taa    1794
Glu Phe His Val Asn Asp Gly Ser Val Ser Ile Tyr Val Gln Lys
    470                 475                 480

ggtaataaaa aaacacctcc aagctgagtg cgggtatcag cttggaggtg cgtttatttt 1854

ttcagccgta tgacaaggtc ggcatcaggt gtgacaaata cggtatgctg gctgtcatag 1914

gtgacaaatc cgggttttgc gccgtttggc tttttcacat gtctgatttt tgtataatca 1974

acaggcacgg agccggaatc tttcgccttg gaaaaataag cggcgatcgt agctgcttcc 2034

aatatggatt gttcatcggg atcgctgctt ttaatcacaa cgtgggatcc            2084


<210> 10
<211> 483
<212> PRT
<213> Bacillus amyloliquefaciens

<400> 10
Val Asn Gly Thr Leu Met Gln Tyr Phe Glu Trp Tyr Thr Pro Asn Asp
1               5                   10                  15

Gly Gln His Trp Lys Arg Leu Gln Asn Asp Ala Glu His Leu Ser Asp
            20                  25                  30

Ile Gly Ile Thr Ala Val Trp Ile Pro Pro Ala Tyr Lys Gly Leu Ser
        35                  40                  45

Gln Ser Asp Asn Gly Tyr Gly Pro Tyr Asp Leu Tyr Asp Leu Gly Glu
    50                  55                  60

Phe Gln Gln Lys Gly Thr Val Arg Thr Lys Tyr Gly Thr Lys Ser Glu
65                  70                  75                  80

Leu Gln Asp Ala Ile Gly Ser Leu His Ser Arg Asn Val Gln Val Tyr
                85                  90                  95

Gly Asp Val Val Leu Asn His Lys Ala Gly Ala Asp Ala Thr Glu Asp
            100                 105                 110

Val Thr Ala Val Glu Val Asn Pro Ala Asn Arg Asn Gln Glu Thr Ser
        115                 120                 125

Glu Glu Tyr Gln Ile Lys Ala Trp Thr Asp Phe Arg Phe Pro Gly Arg
    130                 135                 140

Gly Asn Thr Tyr Ser Asp Phe Lys Trp His Trp Tyr His Phe Asp Gly
145                 150                 155                 160

Ala Asp Trp Asp Glu Ser Arg Lys Ile Ser Arg Ile Phe Lys Phe Arg
                165                 170                 175

Gly Glu Gly Lys Ala Trp Asp Trp Glu Val Ser Ser Glu Asn Gly Asn
            180                 185                 190

Tyr Asp Tyr Leu Met Tyr Ala Asp Val Asp Tyr Asp His Pro Asp Val
        195                 200                 205

Val Ala Glu Thr Lys Lys Trp Gly Ile Trp Tyr Ala Asn Glu Leu Ser
    210                 215                 220

Leu Asp Gly Phe Arg Ile Asp Ala Ala Lys His Ile Lys Phe Ser Phe
225                 230                 235                 240

Leu Arg Asp Trp Val Gln Ala Val Arg Gln Ala Thr Gly Lys Glu Met
                245                 250                 255

Phe Thr Val Ala Glu Tyr Trp Gln Asn Asn Ala Gly Lys Leu Glu Asn
            260                 265                 270

Tyr Leu Asn Lys Thr Ser Phe Asn Gln Ser Val Phe Asp Val Pro Leu
        275                 280                 285

His Phe Asn Leu Gln Ala Ala Ser Ser Gln Gly Gly Gly Tyr Asp Met
    290                 295                 300

Arg Arg Leu Leu Asp Gly Thr Val Val Ser Arg His Pro Glu Lys Ala
305                 310                 315                 320

Val Thr Phe Val Glu Asn His Asp Thr Gln Pro Gly Gln Ser Leu Glu
                325                 330                 335

Ser Thr Val Gln Thr Trp Phe Lys Pro Leu Ala Tyr Ala Phe Ile Leu
            340                 345                 350

Thr Arg Glu Ser Gly Tyr Pro Gln Val Phe Tyr Gly Asp Met Tyr Gly
        355                 360                 365

Thr Lys Gly Thr Ser Pro Lys Glu Ile Pro Ser Leu Lys Asp Asn Ile
    370                 375                 380

Glu Pro Ile Leu Lys Ala Arg Lys Glu Tyr Ala Tyr Gly Pro Gln His
385                 390                 395                 400

Asp Tyr Ile Asp His Pro Asp Val Ile Gly Trp Thr Arg Glu Gly Asp
                405                 410                 415

Ser Ser Ala Ala Lys Ser Gly Leu Ala Ala Leu Ile Thr Asp Gly Pro
            420                 425                 430

Gly Gly Ser Lys Arg Met Tyr Ala Gly Leu Lys Asn Ala Gly Glu Thr
        435                 440                 445

Trp Tyr Asp Ile Thr Gly Asn Arg Ser Asp Thr Val Lys Ile Gly Ser
    450                 455                 460

Asp Gly Trp Gly Glu Phe His Val Asn Asp Gly Ser Val Ser Ile Tyr
465                 470                 475                 480

Val Gln Lys

<210> 11
<211> 1458
<212> DNA
<213> Bacillus sp.

<220>
<221> CDS
<222> (1)..(1458)
<223> AA560

<400> 11
cac cat aat ggt acg aac ggc aca atg atg cag tac ttt gaa tgg tat     48
His His Asn Gly Thr Asn Gly Thr Met Met Gln Tyr Phe Glu Trp Tyr
1               5                   10                  15

cta cca aat gac gga aac cat tgg aat aga tta agg tct gat gca agt     96
Leu Pro Asn Asp Gly Asn His Trp Asn Arg Leu Arg Ser Asp Ala Ser
            20                  25                  30

aac cta aaa gat aaa ggg atc tca gcg gtt tgg att cct cct gca tgg     144
Asn Leu Lys Asp Lys Gly Ile Ser Ala Val Trp Ile Pro Pro Ala Trp
        35                  40                  45

aag ggt gcc tct caa aat gat gtg ggg tat ggt gct tat gat ctg tat     192
Lys Gly Ala Ser Gln Asn Asp Val Gly Tyr Gly Ala Tyr Asp Leu Tyr
    50                  55                  60

gat tta gga gaa ttc aat caa aaa gga acc att cgt aca aaa tat gga     240
Asp Leu Gly Glu Phe Asn Gln Lys Gly Thr Ile Arg Thr Lys Tyr Gly
65                  70                  75                  80

acg cgc aat cag tta caa gct gca gtt aac gcc ttg aaa agt aat gga     288
Thr Arg Asn Gln Leu Gln Ala Ala Val Asn Ala Leu Lys Ser Asn Gly
                85                  90                  95

att caa gtg tat ggc gat gtt gta atg aat cat aaa ggg gga gca gac     336
Ile Gln Val Tyr Gly Asp Val Val Met Asn His Lys Gly Gly Ala Asp
            100                 105                 110

gct acc gaa atg gtt agg gca gtt gaa gta aac ccg aat aat aga aat     384
Ala Thr Glu Met Val Arg Ala Val Glu Val Asn Pro Asn Asn Arg Asn
        115                 120                 125

caa gaa gtg tcc ggt gaa tat aca att gag gct tgg aca aag ttt gac     432
Gln Glu Val Ser Gly Glu Tyr Thr Ile Glu Ala Trp Thr Lys Phe Asp
    130                 135                 140

ttt cca gga cga ggt aat act cat tca aac ttc aaa tgg aga tgg tat     480
Phe Pro Gly Arg Gly Asn Thr His Ser Asn Phe Lys Trp Arg Trp Tyr
145                 150                 155                 160

cac ttt gat gga gta gat tgg gat cag tca cgt aag ctg aac aat cga     528
His Phe Asp Gly Val Asp Trp Asp Gln Ser Arg Lys Leu Asn Asn Arg
                165                 170                 175

att tat aaa ttt aga ggt gat gga aaa ggg tgg gat tgg gaa gtc gat     576
Ile Tyr Lys Phe Arg Gly Asp Gly Lys Gly Trp Asp Trp Glu Val Asp
            180                 185                 190

aca gaa aac ggt aac tat gat tac cta atg tat gca gat att gac atg     624
Thr Glu Asn Gly Asn Tyr Asp Tyr Leu Met Tyr Ala Asp Ile Asp Met
        195                 200                 205

gat cac cca gag gta gtg aat gag cta aga aat tgg ggt gtt tgg tat     672
Asp His Pro Glu Val Val Asn Glu Leu Arg Asn Trp Gly Val Trp Tyr
    210                 215                 220

acg aat aca tta ggc ctt gat ggt ttt aga ata gat gca gta aaa cat     720
Thr Asn Thr Leu Gly Leu Asp Gly Phe Arg Ile Asp Ala Val Lys His
225                 230                 235                 240

ata aaa tac agc ttt act cgt gat tgg att aat cat gtt aga agt gca     768
Ile Lys Tyr Ser Phe Thr Arg Asp Trp Ile Asn His Val Arg Ser Ala
            245                 250                 255

act ggc aaa aat atg ttt gcg gtt gcg gaa ttt tgg aaa aat gat tta     816
Thr Gly Lys Asn Met Phe Ala Val Ala Glu Phe Trp Lys Asn Asp Leu
        260                 265                 270

ggt gct att gaa aac tat tta aac aaa aca aac tgg aac cat tca gtc     864
Gly Ala Ile Glu Asn Tyr Leu Asn Lys Thr Asn Trp Asn His Ser Val
    275                 280                 285

ttt gat gtt ccg ctg cac tat aac ctc tat aat gct tca aaa agc gga     912
Phe Asp Val Pro Leu His Tyr Asn Leu Tyr Asn Ala Ser Lys Ser Gly
    290                 295                 300

ggg aat tat gat atg agg caa ata ttt aat ggt aca gtc gtg caa aga     960
Gly Asn Tyr Asp Met Arg Gln Ile Phe Asn Gly Thr Val Val Gln Arg
305                 310                 315                 320

cat cca atg cat gct gtt aca ttt gtt gat aat cat gat tcg caa cct     1008
His Pro Met His Ala Val Thr Phe Val Asp Asn His Asp Ser Gln Pro
            325                 330                 335

gaa gaa gct tta gag tct ttt gtt gaa gaa tgg ttc aaa cca tta gcg     1056
Glu Glu Ala Leu Glu Ser Phe Val Glu Glu Trp Phe Lys Pro Leu Ala
        340                 345                 350

tat gct ttg aca tta aca cgt gaa caa ggc tac cct tct gta ttt tat     1104
Tyr Ala Leu Thr Leu Thr Arg Glu Gln Gly Tyr Pro Ser Val Phe Tyr
    355                 360                 365

gga gat tat tat ggc att cca acg cat ggt gta cca gcg atg aaa tcg     1152
Gly Asp Tyr Tyr Gly Ile Pro Thr His Gly Val Pro Ala Met Lys Ser
    370                 375                 380

aaa att gac ccg att cta gaa gcg cgt caa aag tat gca tat gga aga     1200
Lys Ile Asp Pro Ile Leu Glu Ala Arg Gln Lys Tyr Ala Tyr Gly Arg
385                 390                 395                 400

caa aat gac tac tta gac cat cat aat atc atc ggt tgg aca cgt gaa     1248
Gln Asn Asp Tyr Leu Asp His His Asn Ile Ile Gly Trp Thr Arg Glu
            405                 410                 415

ggg aat aca gca cac ccc aac tcc ggt tta gct act atc atg tcc gat     1296
Gly Asn Thr Ala His Pro Asn Ser Gly Leu Ala Thr Ile Met Ser Asp
        420                 425                 430

ggg gca gga gga aat aag tgg atg ttt gtt ggg cgt aat aaa gct ggt     1344
Gly Ala Gly Gly Asn Lys Trp Met Phe Val Gly Arg Asn Lys Ala Gly
    435                 440                 445

caa gtt tgg acc gat atc act gga aat cgt gca ggt act gtt acg att     1392
Gln Val Trp Thr Asp Ile Thr Gly Asn Arg Ala Gly Thr Val Thr Ile
    450                 455                 460

aat gct gat gga tgg ggt aat ttt tct gta aat gga gga tca gtt tct     1440
Asn Ala Asp Gly Trp Gly Asn Phe Ser Val Asn Gly Gly Ser Val Ser
465                 470                 475                 480

att tgg gta aac aaa taa                                             1458
Ile Trp Val Asn Lys
            485

<210> 12
<211> 485
<212> PRT
<213> Bacillus sp.

<400> 12
His His Asn Gly Thr Asn Gly Thr Met Met Gln Tyr Phe Glu Trp Tyr
1               5                   10                  15

Leu Pro Asn Asp Gly Asn His Trp Asn Arg Leu Arg Ser Asp Ala Ser
            20                  25                  30

Asn Leu Lys Asp Lys Gly Ile Ser Ala Val Trp Ile Pro Pro Ala Trp
        35                  40                  45

Lys Gly Ala Ser Gln Asn Asp Val Gly Tyr Gly Ala Tyr Asp Leu Tyr
    50                  55                  60

Asp Leu Gly Glu Phe Asn Gln Lys Gly Thr Ile Arg Thr Lys Tyr Gly
65                  70                  75                  80

Thr Arg Asn Gln Leu Gln Ala Ala Val Asn Ala Leu Lys Ser Asn Gly
                85                  90                  95

Ile Gln Val Tyr Gly Asp Val Val Met Asn His Lys Gly Gly Ala Asp
            100                 105                 110

Ala Thr Glu Met Val Arg Ala Val Glu Val Asn Pro Asn Asn Arg Asn
        115                 120                 125

Gln Glu Val Ser Gly Glu Tyr Thr Ile Glu Ala Trp Thr Lys Phe Asp
    130                 135                 140

Phe Pro Gly Arg Gly Asn Thr His Ser Asn Phe Lys Trp Arg Trp Tyr
145                 150                 155                 160

His Phe Asp Gly Val Asp Trp Asp Gln Ser Arg Lys Leu Asn Asn Arg
                165                 170                 175

Ile Tyr Lys Phe Arg Gly Asp Gly Lys Gly Trp Asp Trp Glu Val Asp
            180                 185                 190

Thr Glu Asn Gly Asn Tyr Asp Tyr Leu Met Tyr Ala Asp Ile Asp Met
        195                 200                 205

Asp His Pro Glu Val Val Asn Glu Leu Arg Asn Trp Gly Val Trp Tyr
    210                 215                 220

Thr Asn Thr Leu Gly Leu Asp Gly Phe Arg Ile Asp Ala Val Lys His
225                 230                 235                 240

Ile Lys Tyr Ser Phe Thr Arg Asp Trp Ile Asn His Val Arg Ser Ala
                245                 250                 255

Thr Gly Lys Asn Met Phe Ala Val Ala Glu Phe Trp Lys Asn Asp Leu
            260                 265                 270

Gly Ala Ile Glu Asn Tyr Leu Asn Lys Thr Asn Trp Asn His Ser Val
        275                 280                 285

Phe Asp Val Pro Leu His Tyr Asn Leu Tyr Asn Ala Ser Lys Ser Gly
    290                 295                 300

Gly Asn Tyr Asp Met Arg Gln Ile Phe Asn Gly Thr Val Val Gln Arg
305                 310                 315                 320

His Pro Met His Ala Val Thr Phe Val Asp Asn His Asp Ser Gln Pro
                325                 330                 335

Glu Glu Ala Leu Glu Ser Phe Val Glu Glu Trp Phe Lys Pro Leu Ala
            340                 345                 350

Tyr Ala Leu Thr Leu Thr Arg Glu Gln Gly Tyr Pro Ser Val Phe Tyr
        355                 360                 365

Gly Asp Tyr Tyr Gly Ile Pro Thr His Gly Val Pro Ala Met Lys Ser
    370                 375                 380

Lys Ile Asp Pro Ile Leu Glu Ala Arg Gln Lys Tyr Ala Tyr Gly Arg
385                 390                 395                 400

Gln Asn Asp Tyr Leu Asp His His Asn Ile Ile Gly Trp Thr Arg Glu
                405                 410                 415

Gly Asn Thr Ala His Pro Asn Ser Gly Leu Ala Thr Ile Met Ser Asp
            420                 425                 430

Gly Ala Gly Gly Asn Lys Trp Met Phe Val Gly Arg Asn Lys Ala Gly
        435                 440                 445

Gln Val Trp Thr Asp Ile Thr Gly Asn Arg Ala Gly Thr Val Thr Ile
    450                 455                 460

Asn Ala Asp Gly Trp Gly Asn Phe Ser Val Asn Gly Gly Ser Val Ser
465                 470                 475                 480

Ile Trp Val Asn Lys
                485


<210> 13
<211> 485
<212> PRT
<213> Bacillus sp. 707

<400> 13
His His Asn Gly Thr Asn Gly Thr Met Met Gln Tyr Phe Glu Trp Tyr
  1               5                  10                  15

Leu Pro Asn Asp Gly Asn His Trp Asn Arg Leu Asn Ser Asp Ala Ser
             20                  25                  30

Asn Leu Lys Ser Lys Gly Ile Thr Ala Val Trp Ile Pro Pro Ala Trp
         35                  40                  45

Lys Gly Ala Ser Gln Asn Asp Val Gly Tyr Gly Ala Tyr Asp Leu Tyr
     50                  55                  60

Asp Leu Gly Glu Phe Asn Gln Lys Gly Thr Val Arg Thr Lys Tyr Gly
 65                  70                  75                  80

Thr Arg Ser Gln Leu Gln Ala Ala Val Thr Ser Leu Lys Asn Asn Gly
                 85                  90                  95

Ile Gln Val Tyr Gly Asp Val Val Met Asn His Lys Gly Gly Ala Asp
            100                 105                 110

Ala Thr Glu Met Val Arg Ala Val Glu Val Asn Pro Asn Asn Arg Asn
        115                 120                 125

Gln Glu Val Thr Gly Glu Tyr Thr Ile Glu Ala Trp Thr Arg Phe Asp
    130                 135                 140

Phe Pro Gly Arg Gly Asn Thr His Ser Ser Phe Lys Trp Arg Trp Tyr
145                 150                 155                 160

His Phe Asp Gly Val Asp Trp Asp Gln Ser Arg Arg Leu Asn Asn Arg
                165                 170                 175

Ile Tyr Lys Phe Arg Gly His Gly Lys Ala Trp Asp Trp Glu Val Asp
            180                 185                 190

Thr Glu Asn Gly Asn Tyr Asp Tyr Leu Met Tyr Ala Asp Ile Asp Met
        195                 200                 205

Asp His Pro Glu Val Val Asn Glu Leu Arg Asn Trp Gly Val Trp Tyr
    210                 215                 220

Thr Asn Thr Leu Gly Leu Asp Gly Phe Arg Ile Asp Ala Val Lys His
225                 230                 235                 240

Ile Lys Tyr Ser Phe Thr Arg Asp Trp Ile Asn His Val Arg Ser Ala
                245                 250                 255

Thr Gly Lys Asn Met Phe Ala Val Ala Glu Phe Trp Lys Asn Asp Leu
            260                 265                 270

Gly Ala Ile Glu Asn Tyr Leu Gln Lys Thr Asn Trp Asn His Ser Val
        275                 280                 285

Phe Asp Val Pro Leu His Tyr Asn Leu Tyr Asn Ala Ser Lys Ser Gly
    290                 295                 300

Gly Asn Tyr Asp Met Arg Asn Ile Phe Asn Gly Thr Val Val Gln Arg
305                 310                 315                 320

His Pro Ser His Ala Val Thr Phe Val Asp Asn His Asp Ser Gln Pro
                325                 330                 335

Glu Glu Ala Leu Glu Ser Phe Val Glu Glu Trp Phe Lys Pro Leu Ala
            340                 345                 350

Tyr Ala Leu Thr Leu Thr Arg Glu Gln Gly Tyr Pro Ser Val Phe Tyr
        355                 360                 365

Gly Asp Tyr Tyr Gly Ile Pro Thr His Gly Val Pro Ala Met Arg Ser
    370                 375                 380

Lys Ile Asp Pro Ile Leu Glu Ala Arg Gln Lys Tyr Ala Tyr Gly Lys
385                 390                 395                 400

Gln Asn Asp Tyr Leu Asp His His Asn Ile Ile Gly Trp Thr Arg Glu
                405                 410                 415

Gly Asn Thr Ala His Pro Asn Ser Gly Leu Ala Thr Ile Met Ser Asp
            420                 425                 430

Gly Ala Gly Gly Ser Lys Trp Met Phe Val Gly Arg Asn Lys Ala Gly
        435                 440                 445

Gln Val Trp Ser Asp Ile Thr Gly Asn Arg Thr Gly Thr Val Thr Ile
    450                 455                 460

Asn Ala Asp Gly Trp Gly Asn Phe Ser Val Asn Gly Gly Ser Val Ser
465                 470                 475                 480

Ile Trp Val Asn Lys
                485

<210> 14
<211> 27
<212> DNA
<213> Artificial Sequence
<220>
<223> Primer 22149
<400> 14
CGATTGCTGA CGCTGTTATT TGCG

24

<210> 15
<211> 29
<212> DNA
<213> Artificial Sequence
<220>
<223> Primer 24814
<400> 15
GATCACCCGC GATACCGTC
29

<210> 16
<211> 31
<212> DNA
<213> Artificial Sequence
<220>
<223> Primer #24
<400> 16
GAATGTATGT CGGCCGGCAA AACGCCGGTG A
31
<210> 17
<211> 30
<212> DNA
<213> Artificial Sequence
<220>
<223> Primer #27
<400> 17
GCCGCCGCTG CTGCAGAATG AGGCAGCAAG
30

<210> 18
<211> 48
<212> DNA
<213> Artificial Sequence
<220>
<223> Primer #312
<400> 18
CCCGAAAGCT GAACCGCATC TATAGGTTTC AAGGGAAGAC TTGGGATT 48

<210> 19
<211> 23
<212> DNA
<213> Artificial Sequence
<220>
<223> Primer #290
<400> 19
AGGATGGTCA TAATCAAAGT CGG
23

<210> 20
<211> 52
<212> DNA
<213> Artificial Sequence
<220>
<223> Primer #313
<400> 20
CCGACTTTGA TTATGACCAT CCTGTTGTCG TAGCAGAGAT TAAGAGATGG GG 52

<210> 21
<211> 45
<212> DNA
<213> Artificial Sequence
<220>
<223> Primer #314
<400> 21
CGACAATGTC ATGGTGGTCG AAAAAATCAT GCTGTGCTCC GTACG 45

<210> 22

<211> 23
<212> DNA
<213> Artificial Sequence
<220>
<223> Primer #296
<400> 22
TTTCGACCAC CATGACATTG TCG
23

<210> 23
<211> 24
<212> DNA
<213> Artificial Sequence
<220>
<223> Primer #305
<400> 23
TATAGATGCG GTTCAGCTTT CGGG
24

<210> 24
<211> 1650
<212> DNA
<213> Bacillus sp.
<220>
<221> CDS
<222> (65)..(1567)
<220>
<221> mat_peptide
<222> (128)..()
<220>
<221> sig_peptide
<222> (65)..(128)

<400> 24
cttgaatcat tatttaaagc tggttatgat atatgtaagc gttatcatta aaaggaggta 60

tttg atg aaa aga tgg gta gta gca atg ctg gca gtg tta ttt tta ttt 109
Met Lys Arg Trp Val Val Ala Met Leu Ala Val Leu Phe Leu Phe
-20 -15 -10

cct tcg gta gta gtt gca gat ggc ttg aat gga acg atg atg cag tat 157
Pro Ser Val Val Val Ala Asp Gly Leu Asn Gly Thr Met Met Gln Tyr
-5 -1 1 5 10

tat gag tgg cat cta gag aat gat ggg caa cac tgg aat cgg ttg cat 205
Tyr Glu Trp His Leu Glu Asn Asp Gly Gln His Trp Asn Arg Leu His
15 20 25

gat gat gcc gaa gct tta agt aat gcg ggt att aca gct att tgg ata 253
Asp Asp Ala Glu Ala Leu Ser Asn Ala Gly Ile Thr Ala Ile Trp Ile
30 35 40

ccc cca gcc tac aaa gga aat agt cag gct gat gtt ggg tat ggt gca 301
Pro Pro Ala Tyr Lys Gly Asn Ser Gln Ala Asp Val Gly Tyr Gly Ala
45 50 55

tac gac ctt tat gat tta ggg gag ttt aat caa aaa ggt acc gtt cga 349
Tyr Asp Leu Tyr Asp Leu Gly Glu Phe Asn Gln Lys Gly Thr Val Arg
60 65 70

acg aaa tac ggg aca aag gct cag ctt gag cga gct ata ggg tcc cta 397
Thr Lys Tyr Gly Thr Lys Ala Gln Leu Glu Arg Ala Ile Gly Ser Leu
75 80 85 90

aag tcg aat gat atc aat gtt tat ggg gat gtc gta atg aat cat aaa 445
Lys Ser Asn Asp Ile Asn Val Tyr Gly Asp Val Val Met Asn His Lys
95 100 105

tta gga gct gat ttc acg gag gca gtg caa gct gtt caa gta aat cct 493

Leu Gly Ala Asp Phe Thr Glu Ala Val Gln Ala Val Gln Val Asn Pro
            110                 115                 120

tcg aac cgt tgg cag gat att tca ggt gtc tac acg att gat gca tgg      541
Ser Asn Arg Trp Gln Asp Ile Ser Gly Val Tyr Thr Ile Asp Ala Trp
        125                 130                 135

acg gga ttt gac ttt cca ggg cgc aac aat gcc tat tcc gat ttt aaa      589
Thr Gly Phe Asp Phe Pro Gly Arg Asn Asn Ala Tyr Ser Asp Phe Lys
    140                 145                 150

tgg aga tgg ttc cat ttt aat ggc gtt gac tgg gat caa cgc tat caa      637
Trp Arg Trp Phe His Phe Asn Gly Val Asp Trp Asp Gln Arg Tyr Gln
155                 160                 165                 170

gaa aac cat ctt ttt cgc ttt gca aat acg aac tgg aac tgg cga gtg      685
Glu Asn His Leu Phe Arg Phe Ala Asn Thr Asn Trp Asn Trp Arg Val
                175                 180                 185

gat gaa gag aat ggt aat tat gac tat tta tta gga tcg aac att gac      733
Asp Glu Glu Asn Gly Asn Tyr Asp Tyr Leu Leu Gly Ser Asn Ile Asp
            190                 195                 200

ttt agc cac cca gag gtt caa gag gaa tta aag gat tgg ggg agc tgg      781
Phe Ser His Pro Glu Val Gln Glu Glu Leu Lys Asp Trp Gly Ser Trp
        205                 210                 215

ttt acg gat gag cta gat tta gat ggg tat cga ttg gat gct att aag      829
Phe Thr Asp Glu Leu Asp Leu Asp Gly Tyr Arg Leu Asp Ala Ile Lys
    220                 225                 230

cat att cca ttc tgg tat acg tca gat tgg gtt agg cat cag cga agt      877
His Ile Pro Phe Trp Tyr Thr Ser Asp Trp Val Arg His Gln Arg Ser
235                 240                 245                 250

gaa gca gac caa gat tta ttt gtc gta ggg gag tat tgg aag gat gac      925
Glu Ala Asp Gln Asp Leu Phe Val Val Gly Glu Tyr Trp Lys Asp Asp
                255                 260                 265

gta ggt gct ctc gaa ttt tat tta gat gaa atg aat tgg gag atg tct      973
Val Gly Ala Leu Glu Phe Tyr Leu Asp Glu Met Asn Trp Glu Met Ser
            270                 275                 280

cta ttc gat gtt ccg ctc aat tat aat ttt tac cgg gct tca aag caa     1021
Leu Phe Asp Val Pro Leu Asn Tyr Asn Phe Tyr Arg Ala Ser Lys Gln
        285                 290                 295

ggc gga agc tat gat atg cgt aat att tta cga gga tct tta gta gaa     1069
Gly Gly Ser Tyr Asp Met Arg Asn Ile Leu Arg Gly Ser Leu Val Glu
    300                 305                 310

gca cat ccg att cat gca gtt acg ttt gtt gat aat cat gat act cag     1117
Ala His Pro Ile His Ala Val Thr Phe Val Asp Asn His Asp Thr Gln
315                 320                 325                 330

cca gga gag tca tta gaa tca tgg gtc gct gat tgg ttt aag cca ctt     1165
Pro Gly Glu Ser Leu Glu Ser Trp Val Ala Asp Trp Phe Lys Pro Leu
                335                 340                 345

gct tat gcg aca atc ttg acg cgt gaa ggt ggt tat cca aat gta ttt     1213
Ala Tyr Ala Thr Ile Leu Thr Arg Glu Gly Gly Tyr Pro Asn Val Phe
            350                 355                 360

tac ggt gac tac tat ggg att cct aac gat aac att tca gct aag aag     1261
Tyr Gly Asp Tyr Tyr Gly Ile Pro Asn Asp Asn Ile Ser Ala Lys Lys
        365                 370                 375

gat atg att gat gag ttg ctt gat gca cgt caa aat tac gca tat ggc     1309
Asp Met Ile Asp Glu Leu Leu Asp Ala Arg Gln Asn Tyr Ala Tyr Gly

380                 385                 390

aca caa cat gac tat ttt gat cat tgg gat atc gtt gga tgg aca aga      1357
Thr Gln His Asp Tyr Phe Asp His Trp Asp Ile Val Gly Trp Thr Arg
395                 400                 405                 410

gaa ggt aca tcc tca cgt cct aat tcg ggt ctt gct act att atg tcc      1405
Glu Gly Thr Ser Ser Arg Pro Asn Ser Gly Leu Ala Thr Ile Met Ser
                415                 420                 425

aat ggt cct gga gga tca aaa tgg atg tac gta gga cag caa cat gca      1453
Asn Gly Pro Gly Gly Ser Lys Trp Met Tyr Val Gly Gln Gln His Ala
            430                 435                 440

gga caa acg tgg aca gat tta act ggc aat cac gcg gcg tcg gtt acg      1501
Gly Gln Thr Trp Thr Asp Leu Thr Gly Asn His Ala Ala Ser Val Thr
        445                 450                 455

att aat ggt gat ggc tgg ggc gaa ttc ttt aca aat gga gga tct gta      1549
Ile Asn Gly Asp Gly Trp Gly Glu Phe Phe Thr Asn Gly Gly Ser Val
    460                 465                 470

tcc gtg tat gtg aac caa taataaaaag ccttgagaag ggattcctcc             1597
Ser Val Tyr Val Asn Gln
475                 480

ctaactcaag gctttcttta tgtcgtttag ctcaacgctt ctacgaagct tta           1650


<210>  25
<211>  501
<212>  PRT
<213>  Bacillus sp.
<400>  25
Met Lys Arg Trp Val Val Ala Met Leu Ala Val Leu Phe Leu Phe Pro
    -20                 -15                 -10

Ser Val Val Val Ala Asp Gly Leu Asn Gly Thr Met Met Gln Tyr Tyr
-5                  -1  1               5                   10

Glu Trp His Leu Glu Asn Asp Gly Gln His Trp Asn Arg Leu His Asp
            15                  20                  25

Asp Ala Glu Ala Leu Ser Asn Ala Gly Ile Thr Ala Ile Trp Ile Pro
        30                  35                  40

Pro Ala Tyr Lys Gly Asn Ser Gln Ala Asp Val Gly Tyr Gly Ala Tyr
    45                  50                  55

Asp Leu Tyr Asp Leu Gly Glu Phe Asn Gln Lys Gly Thr Val Arg Thr
60                  65                  70                  75

Lys Tyr Gly Thr Lys Ala Gln Leu Glu Arg Ala Ile Gly Ser Leu Lys
                80                  85                  90

Ser Asn Asp Ile Asn Val Tyr Gly Asp Val Val Met Asn His Lys Leu
            95                  100                 105

Gly Ala Asp Phe Thr Glu Ala Val Gln Ala Val Gln Val Asn Pro Ser
        110                 115                 120

Asn Arg Trp Gln Asp Ile Ser Gly Val Tyr Thr Ile Asp Ala Trp Thr
    125                 130                 135

Gly Phe Asp Phe Pro Gly Arg Asn Asn Ala Tyr Ser Asp Phe Lys Trp
140                 145                 150                 155

Arg Trp Phe His Phe Asn Gly Val Asp Trp Asp Gln Arg Tyr Gln Glu
                160                 165                 170

Asn His Leu Phe Arg Phe Ala Asn Thr Asn Trp Asn Trp Arg Val Asp
            175                 180                 185

Glu Glu Asn Gly Asn Tyr Asp Tyr Leu Leu Gly Ser Asn Ile Asp Phe
        190                 195                 200

Ser His Pro Glu Val Gln Glu Glu Leu Lys Asp Trp Gly Ser Trp Phe
    205                 210                 215

Thr Asp Glu Leu Asp Leu Asp Gly Tyr Arg Leu Asp Ala Ile Lys His
220                 225                 230                 235

Ile Pro Phe Trp Tyr Thr Ser Asp Trp Val Arg His Gln Arg Ser Glu
                240                 245                 250

Ala Asp Gln Asp Leu Phe Val Val Gly Glu Tyr Trp Lys Asp Asp Val
            255                 260                 265

Gly Ala Leu Glu Phe Tyr Leu Asp Glu Met Asn Trp Glu Met Ser Leu
        270                 275                 280

Phe Asp Val Pro Leu Asn Tyr Asn Phe Tyr Arg Ala Ser Lys Gln Gly
    285                 290                 295

Gly Ser Tyr Asp Met Arg Asn Ile Leu Arg Gly Ser Leu Val Glu Ala
300                 305                 310                 315

His Pro Ile His Ala Val Thr Phe Val Asp Asn His Asp Thr Gln Pro
                320                 325                 330

Gly Glu Ser Leu Glu Ser Trp Val Ala Asp Trp Phe Lys Pro Leu Ala
            335                 340                 345

Tyr Ala Thr Ile Leu Thr Arg Glu Gly Gly Tyr Pro Asn Val Phe Tyr
        350                 355                 360

Gly Asp Tyr Tyr Gly Ile Pro Asn Asp Asn Ile Ser Ala Lys Lys Asp
    365                 370                 375

Met Ile Asp Glu Leu Leu Asp Ala Arg Gln Asn Tyr Ala Tyr Gly Thr
380                 385                 390                 395

Gln His Asp Tyr Phe Asp His Trp Asp Ile Val Gly Trp Thr Arg Glu

400                 405                 410

Gly Thr Ser Ser Arg Pro Asn Ser Gly Leu Ala Thr Ile Met Ser Asn
            415                 420                 425

Gly Pro Gly Gly Ser Lys Trp Met Tyr Val Gly Gln Gln His Ala Gly
        430                 435                 440

Gln Thr Trp Thr Asp Leu Thr Gly Asn His Ala Ala Ser Val Thr Ile
    445                 450                 455

Asn Gly Asp Gly Trp Gly Glu Phe Phe Thr Asn Gly Gly Ser Val Ser
460                 465                 470                 475

Val Tyr Val Asn Gln
                480

<210>  26
<211>  1745
<212>  DNA
<213>  Bacillus sp.
<220>
<221>  CDS
<222>  (190)..(1692)
<220>
<221>  mat_peptide
<222>  (253)..()
<220>
<221>  sig_peptide
<222>  (190)..(253)
<400>  26
aactaagtaa catcgattca ggataaaagt atgcgaaacg atgcgcaaaa ctgcgcaact      60

actagcactc ttcagggact aaaccacctt ttttccaaaa atgacatcat ataaacaaat     120

ttgtctacca atcactattt aaagctgttt atgatatatg taagcgttat cattaaaagg     180

aggtatttg atg aga aga tgg gta gta gca atg ttg gca gtg tta ttt tta     231
          Met Arg Arg Trp Val Val Ala Met Leu Ala Val Leu Phe Leu
              -20                 -15                 -10

ttt cct tcg gta gta gtt gca gat gga ttg aac ggt acg atg atg cag       279
Phe Pro Ser Val Val Val Ala Asp Gly Leu Asn Gly Thr Met Met Gln
        -5                  -1  1               5

tat tat gag tgg cat ttg gaa aac gac ggg cag cat tgg aat cgg ttg       327
Tyr Tyr Glu Trp His Leu Glu Asn Asp Gly Gln His Trp Asn Arg Leu
10                  15                  20                  25

cac gat gat gcc gca gct ttg agt gat gct ggt att aca gct att tgg       375
His Asp Asp Ala Ala Ala Leu Ser Asp Ala Gly Ile Thr Ala Ile Trp
                30                  35                  40

att ccg cca gcc tac aaa ggt aat agt cag gcg gat gtt ggg tac ggt       423
Ile Pro Pro Ala Tyr Lys Gly Asn Ser Gln Ala Asp Val Gly Tyr Gly
            45                  50                  55

gca tac gat ctt tat gat tta gga gag ttc aat caa aag ggt act gtt       471
Ala Tyr Asp Leu Tyr Asp Leu Gly Glu Phe Asn Gln Lys Gly Thr Val
        60                  65                  70

cga acg aaa tac gga act aag gca cag ctt gaa cga gct att ggg tcc       519
Arg Thr Lys Tyr Gly Thr Lys Ala Gln Leu Glu Arg Ala Ile Gly Ser

75                  80                  85

ctt aaa tct aat gat atc aat gta tac gga gat gtc gtg atg aat cat      567
Leu Lys Ser Asn Asp Ile Asn Val Tyr Gly Asp Val Val Met Asn His
90                  95                  100                 105

aaa atg gga gct gat ttt acg gag gca gtg caa gct gtt caa gta aat      615
Lys Met Gly Ala Asp Phe Thr Glu Ala Val Gln Ala Val Gln Val Asn
                110                 115                 120

cca acg aat cgt tgg cag gat att tca ggt gcc tac acg att gat gcg      663
Pro Thr Asn Arg Trp Gln Asp Ile Ser Gly Ala Tyr Thr Ile Asp Ala
            125                 130                 135

tgg acg ggt ttc gac ttt tca ggg cgt aac aac gcc tat tca gat ttt      711
Trp Thr Gly Phe Asp Phe Ser Gly Arg Asn Asn Ala Tyr Ser Asp Phe
        140                 145                 150

aag tgg aga tgg ttc cat ttt aat ggt gtt gac tgg gat cag cgc tat      759
Lys Trp Arg Trp Phe His Phe Asn Gly Val Asp Trp Asp Gln Arg Tyr
    155                 160                 165

caa gaa aat cat att ttc cgc ttt gca aat acg aac tgg aac tgg cga      807
Gln Glu Asn His Ile Phe Arg Phe Ala Asn Thr Asn Trp Asn Trp Arg
170                 175                 180                 185

gtg gat gaa gag aac ggt aat tat gat tac ctg tta gga tcg aat atc      855
Val Asp Glu Glu Asn Gly Asn Tyr Asp Tyr Leu Leu Gly Ser Asn Ile
                190                 195                 200

gac ttt agt cat cca gaa gta caa gat gag ttg aag gat tgg ggt agc      903
Asp Phe Ser His Pro Glu Val Gln Asp Glu Leu Lys Asp Trp Gly Ser
            205                 210                 215

tgg ttt acc gat gag tta gat ttg gat ggt tat cgt tta gat gct att      951
Trp Phe Thr Asp Glu Leu Asp Leu Asp Gly Tyr Arg Leu Asp Ala Ile
        220                 225                 230

aaa cat att cca ttc tgg tat aca tct gat tgg gtt cgg cat cag cgc      999
Lys His Ile Pro Phe Trp Tyr Thr Ser Asp Trp Val Arg His Gln Arg
    235                 240                 245

aac gaa gca gat caa gat tta ttt gtc gta ggg gaa tat tgg aag gat     1047
Asn Glu Ala Asp Gln Asp Leu Phe Val Val Gly Glu Tyr Trp Lys Asp
250                 255                 260                 265

gac gta ggt gct ctc gaa ttt tat tta gat gaa atg aat tgg gag atg     1095
Asp Val Gly Ala Leu Glu Phe Tyr Leu Asp Glu Met Asn Trp Glu Met
                270                 275                 280

tct cta ttc gat gtt cca ctt aat tat aat ttt tac cgg gct tca caa     1143
Ser Leu Phe Asp Val Pro Leu Asn Tyr Asn Phe Tyr Arg Ala Ser Gln
            285                 290                 295

caa ggt gga agc tat gat atg cgt aat att tta cga gga tct tta gta     1191
Gln Gly Gly Ser Tyr Asp Met Arg Asn Ile Leu Arg Gly Ser Leu Val
        300                 305                 310

gaa gcg cat ccg atg cat gca gtt acg ttt gtt gat aat cat gat act     1239
Glu Ala His Pro Met His Ala Val Thr Phe Val Asp Asn His Asp Thr
    315                 320                 325

cag cca ggg gag tca tta gag tca tgg gtt gct gat tgg ttt aag cca     1287
Gln Pro Gly Glu Ser Leu Glu Ser Trp Val Ala Asp Trp Phe Lys Pro
330                 335                 340                 345

ctt gct tat gcg aca att ttg acg cgt gaa ggt ggt tat cca aat gta     1335
Leu Ala Tyr Ala Thr Ile Leu Thr Arg Glu Gly Gly Tyr Pro Asn Val
                350                 355                 360

ttt tac ggt gat tac tat ggg att cct aac gat aac att tca gct aaa 1383
Phe Tyr Gly Asp Tyr Tyr Gly Ile Pro Asn Asp Asn Ile Ser Ala Lys
365 370 375

aaa gat atg att gat gag ctg ctt gat gca cgt caa aat tac gca tat 1431
Lys Asp Met Ile Asp Glu Leu Leu Asp Ala Arg Gln Asn Tyr Ala Tyr
380 385 390

ggc acg cag cat gac tat ttt gat cat tgg gat gtt gta gga tgg act 1479
Gly Thr Gln His Asp Tyr Phe Asp His Trp Asp Val Val Gly Trp Thr
395 400 405

agg gaa gga tct tcc tcc aga cct aat tca ggc ctt gcg act att atg 1527
Arg Glu Gly Ser Ser Ser Arg Pro Asn Ser Gly Leu Ala Thr Ile Met
410 415 420 425

tcg aat gga cct ggt ggt tcc aag tgg atg tat gta gga cgt cag aat 1575
Ser Asn Gly Pro Gly Gly Ser Lys Trp Met Tyr Val Gly Arg Gln Asn
430 435 440

gca gga caa aca tgg aca gat tta act ggt aat aac gga gcg tcc gtt 1623
Ala Gly Gln Thr Trp Thr Asp Leu Thr Gly Asn Asn Gly Ala Ser Val
445 450 455

aca att aat ggc gat gga tgg ggc gaa ttc ttt acg aat gga gga tct 1671
Thr Ile Asn Gly Asp Gly Trp Gly Glu Phe Phe Thr Asn Gly Gly Ser
460 465 470

gta tcc gtg tac gtg aac caa taacaaaaag ccttgagaag ggattcctcc 1722
Val Ser Val Tyr Val Asn Gln
475 480

ctaactcaag gctttcttta tgt 1745

<210> 27
<211> 501
<212> PRT
<213> Bacillus sp.
<400> 27
Met Arg Arg Trp Val Val Ala Met Leu Ala Val Leu Phe Leu Phe Pro
-20 -15 -10

Ser Val Val Val Ala Asp Gly Leu Asn Gly Thr Met Met Gln Tyr Tyr
-5 -1 1 5 10

Glu Trp His Leu Glu Asn Asp Gly Gln His Trp Asn Arg Leu His Asp
15 20 25

Asp Ala Ala Ala Leu Ser Asp Ala Gly Ile Thr Ala Ile Trp Ile Pro
30 35 40

Pro Ala Tyr Lys Gly Asn Ser Gln Ala Asp Val Gly Tyr Gly Ala Tyr
45 50 55

Asp Leu Tyr Asp Leu Gly Glu Phe Asn Gln Lys Gly Thr Val Arg Thr
60 65 70 75

Lys Tyr Gly Thr Lys Ala Gln Leu Glu Arg Ala Ile Gly Ser Leu Lys
80 85 90

Ser Asn Asp Ile Asn Val Tyr Gly Asp Val Val Met Asn His Lys Met

95 100 105

Gly Ala Asp Phe Thr Glu Ala Val Gln Ala Val Gln Val Asn Pro Thr
110 115 120

Asn Arg Trp Gln Asp Ile Ser Gly Ala Tyr Thr Ile Asp Ala Trp Thr
125 130 135

Gly Phe Asp Phe Ser Gly Arg Asn Asn Ala Tyr Ser Asp Phe Lys Trp
140 145 150 155

Arg Trp Phe His Phe Asn Gly Val Asp Trp Asp Gln Arg Tyr Gln Glu
160 165 170

Asn His Ile Phe Arg Phe Ala Asn Thr Asn Trp Asn Trp Arg Val Asp
175 180 185

Glu Glu Asn Gly Asn Tyr Asp Tyr Leu Leu Gly Ser Asn Ile Asp Phe
190 195 200

Ser His Pro Glu Val Gln Asp Glu Leu Lys Asp Trp Gly Ser Trp Phe
205 210 215

Thr Asp Glu Leu Asp Leu Asp Gly Tyr Arg Leu Asp Ala Ile Lys His
220 225 230 235

Ile Pro Phe Trp Tyr Thr Ser Asp Trp Val Arg His Gln Arg Asn Glu
240 245 250

Ala Asp Gln Asp Leu Phe Val Val Gly Glu Tyr Trp Lys Asp Asp Val
255 260 265

Gly Ala Leu Glu Phe Tyr Leu Asp Glu Met Asn Trp Glu Met Ser Leu
270 275 280

Phe Asp Val Pro Leu Asn Tyr Asn Phe Tyr Arg Ala Ser Gln Gln Gly
285 290 295

Gly Ser Tyr Asp Met Arg Asn Ile Leu Arg Gly Ser Leu Val Glu Ala
300 305 310 315

His Pro Met His Ala Val Thr Phe Val Asp Asn His Asp Thr Gln Pro
320 325 330

Gly Glu Ser Leu Glu Ser Trp Val Ala Asp Trp Phe Lys Pro Leu Ala
335 340 345

Tyr Ala Thr Ile Leu Thr Arg Glu Gly Gly Tyr Pro Asn Val Phe Tyr
350 355 360

Gly Asp Tyr Tyr Gly Ile Pro Asn Asp Asn Ile Ser Ala Lys Lys Asp
365 370 375

Met Ile Asp Glu Leu Leu Asp Ala Arg Gln Asn Tyr Ala Tyr Gly Thr
380                 385                 390                 395

Gln His Asp Tyr Phe Asp His Trp Asp Val Val Gly Trp Thr Arg Glu
                400                 405                 410

Gly Ser Ser Ser Arg Pro Asn Ser Gly Leu Ala Thr Ile Met Ser Asn
            415                 420                 425

Gly Pro Gly Gly Ser Lys Trp Met Tyr Val Gly Arg Gln Asn Ala Gly
        430                 435                 440

Gln Thr Trp Thr Asp Leu Thr Gly Asn Asn Gly Ala Ser Val Thr Ile
    445                 450                 455

Asn Gly Asp Gly Trp Gly Glu Phe Phe Thr Asn Gly Gly Ser Val Ser
460                 465                 470                 475

Val Tyr Val Asn Gln
                480

<210>  28
<211>  1920
<212>  DNA
<213>  Bacillus licheniformis
<220>
<221>  CDS
<222>  (421)..(1872)
<400>  28
cggaagattg gaagtacaaa aataagcaaa agattgtcaa tcatgtcatg agccatgcgg      60
gagacggaaa aatcgtctta atgcacgata tttatgcaac gttcgcagat gctgctgaag     120
agattattaa aaagctgaaa gcaaaaggct atcaattggt aactgtatct cagcttgaag     180
aagtgaagaa gcagagaggc tattgaataa atgagtagaa gcgccatatc ggcgcttttc     240
ttttggaaga aaatataggg aaaatggtac ttgttaaaaa ttcggaatat ttatacaaca     300
tcatatgttt cacattgaaa ggggaggaga atcatgaaac aacaaaaacg gctttacgcc     360
cgattgctga cgctgttatt tgcgctcatc ttcttgctgc ctcattctgc agcagcggcg     420
gca aat ctt aat ggg acg ctg atg cag tat ttt gaa tgg tac atg ccc       468
aat gac ggc caa cat tgg agg cgt ttg caa aac gac tcg gca tat ttg       516
gct gaa cac ggt att act gcc gtc tgg att ccc ccg gca tat aag gga       564
acg agc caa gcg gat gtg ggc tac ggt gct tac gac ctt tat gat tta       612
ggg gag ttt cat caa aaa ggg acg gtt cgg aca aag tac ggc aca aaa       660
gga gag ctg caa tct gcg atc aaa agt ctt cat tcc cgc gac att aac      708
gtt tac ggg gat gtg gtc atc aac cac aaa ggc ggc gct gat gcg acc      756
gaa gat gta acc gcg gtt gaa gtc gat ccc gct gac cgc aac cgc gta      804
att tca gga gaa cac cta att aaa gcc tgg aca cat ttt cat ttt ccg      852
ggg cgc ggc agc aca tac agc gat ttt aaa tgg cat tgg tac cat ttt      900
gac gga acc gat tgg gac gag tcc cga aag ctg aac cgc atc tat aag      948
ttt caa gga aag gct tgg gat tgg gaa gtt tcc aat gaa aac ggc aac      996
tat gat tat ttg atg tat gcc gac atc gat tat gac cat cct gat gtc      1044
gca gca gaa att aag aga tgg ggc act tgg tat gcc aat gaa ctg caa      1092
ttg gac ggt ttc cgt ctt gat gct gtc aaa cac att aaa ttt tct ttt      1140
ttg cgg gat tgg gtt aat cat gtc agg gaa aaa acg ggg aag gaa atg      1188
ttt acg gta gct gaa tat tgg cag aat gac ttg ggc gcg ctg gaa aac      1236
tat ttg aac aaa aca aat ttt aat cat tca gtg ttt gac gtg ccg ctt      1284
cat tat cag ttc cat gct gca tcg aca cag gga ggc ggc tat gat atg      1332
agg aaa ttg ctg aac ggt acg gtc gtt tcc aag cat ccg ttg aaa tcg      1380
gtt aca ttt gtc gat aac cat gat aca cag ccg ggg caa tcg ctt gag      1428
tcg act gtc caa aca tgg ttt aag ccg ctt gct tac gct ttt att ctc      1476
aca agg gaa tct gga tac cct cag gtt ttc tac ggg gat atg tac ggg      1524
acg aaa gga gac tcc cag cgc gaa att cct gcc ttg aaa cac aaa att      1572
gaa ccg atc tta aaa gcg aga aaa cag tat gcg tac gga gca cag cat      1620
gat tat ttc gac cac cat gac att gtc ggc tgg aca agg gaa ggc gac      1668
agc tcg gtt gca aat tca ggt ttg gcg gca tta ata aca gac gga ccc      1716
ggt ggg gca aag cga atg tat gtc ggc cgg caa aac gcc ggt gag aca      1764
tgg cat gac att acc gga aac cgt tcg gag ccg gtt gtc atc aat tcg      1812
gaa ggc tgg gga gag ttt cac gta aac ggc ggg tcg gtt tca att tat      1860
gtt caa aga tag aagagcagag aggacggatt tcctgaagga aatccgtttt          1912
tttatttt                                                             1920

## Description

### FIELD OF THE INVENTION

The present invention relates to variants (mutants) of parent Termamyl-like alpha-amylases, which variant has alpha-amylase activity and exhibits an alteration in at least one of the following properties relative to said parent alpha-amylase: stability under, e.g., high temperature and/or low pH conditions, in particular at low calcium concentrations. The variant of the invention are suitable for starch conversion, ethanol production, laundry wash, dish wash, hard surface cleaning, textile desizing, and/or sweetner production.

### BACKGROUND OF THE INVENTION

Alpha-Amylases (alpha-1,4-glucan-4-glucanohydrolases, E.C. 3.2.1.1) constitute a group of enzymes, which catalyze hydrolysis of starch and other linear and branched 1,4-glucosidic oligo- and polysaccharides.

### BRIEF DISCLOSURE OF THE INVENTION

The object of the present invention is to provide Termamyl-like amylases which variants in comparison to the corresponding parent alpha-amylase, i.e., un-mutated alpha-amylase, has alpha-amylase activity and exhibits an alteration in at least one of the following properties relative to said parent alpha-amylase: stability under, e.g., high temperature and/or low pH conditions, in particular at low calcium concentrations.

### Nomenclature

In the present description and claims, the conventional one-letter and three-letter codes for amino acid residues are used. For ease of reference, alpha-amylase variants of the invention are described by use of the following nomenclature: Original amino acid(s): position(s): substituted amino acid(s)

According to this nomenclature, for instance the substitution of alanine for asparagine in position 30 is shown as:
Ala30Asn or A30N
a deletion of alanine in the same position is shown as:
Ala30* or A30*
and insertion of an additional amino acid residue, such as lysine, is shown as:
Ala30AlaLys or A30AK

A deletion of a consecutive stretch of amino acid residues, such as amino acid residues 30-33, is indicated as (30-33)* or Δ(A30-N33).

Where a specific alpha-amylase contains a "deletion" in comparison with other alpha-amylases and an insertion is made in such a position this is indicated as:
*36Asp or *36D
for insertion of an aspartic acid in position 36.
Multiple mutations are separated by plus signs, i.e.:
Ala30Asp + Glu34Ser or A30N+E34S
representing mutations in positions 30 and 34 substituting alanine and glutamic acid for asparagine and serine, respectively.

When one or more alternative amino acid residues may be inserted in a given position it is indicated as
A30N,E or
A30N or A30E

Furthermore, when a position suitable for modification is identified herein without any specific modification being suggested, it is to be understood that any amino acid residue may be substituted for the amino acid residue present in the position. Thus, for instance, when a modification of an alanine in position 30 is mentioned, but not specified, it is to be understood that the alanine may be deleted or substituted for any other amino acid, i.e., any one of:
R,N,D,A,C,Q,E,G,H,I,L,K,M,F,P,S,T,W,Y,V.
Further, "A30X" means any one of the following substitutions:
A30R, A30N, A30D, A30C, A30Q, A30E, A30G, A30H, A30I, A30L, A30K, A30M, A30F, A30P, A30S, A30T, A30W, A30Y, or A30 V; or in short: A30R,N,D,C,Q,E,G,H,I,L,K,M,F,P,S,T,W,Y,V.

If the parent enzyme - used for the numbering - already has the amino acid residue in question suggested for substitution in that position the following nomenclature is used:
"X30N" or "X30N,V" in the case where for instance one or N or V is present in the wildtype.
Thus, it means that other corresponding parent enzymes are substituted to an "Asn" or "Val" in position 30.

### BRIEF DESCRIPTION OF THE DRAWINGS

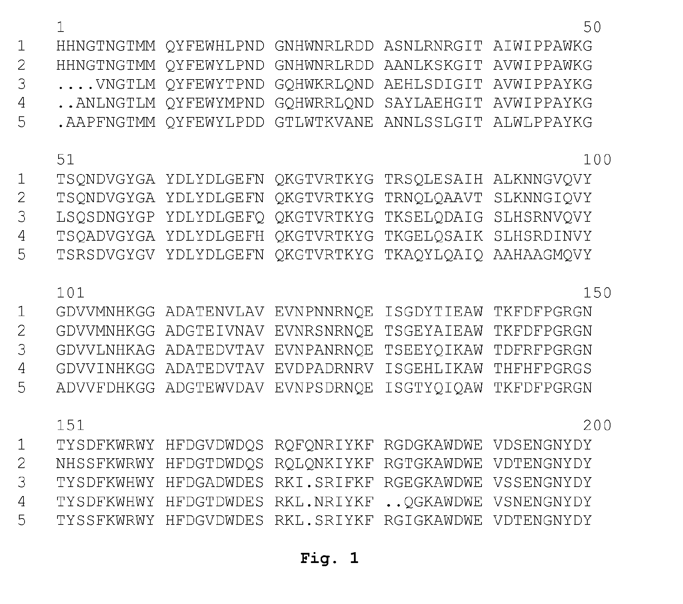

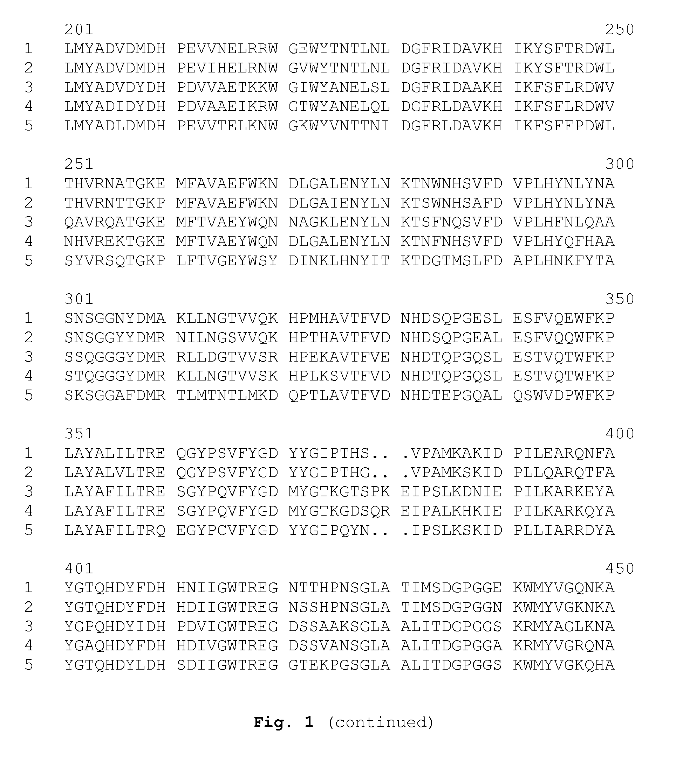

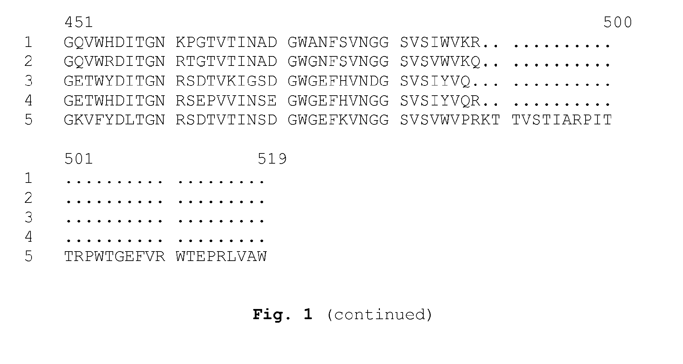

Figure 1 is an alignment of the amino acid sequences of five parent Termamyl-like alpha-amylases. The numbers on the extreme left designate the respective amino acid sequences as follows:
   1: SEQ ID NO: 4 (SP722)
   2: SEQ ID NO: 2 (SP690)
   3: SEQ ID NO: 10 (BAN)
   4: SEQ ID NO: 8 (BLA)
   5: SEQ ID NO: 6 (BSG).

### DETAILED DISCLOSURE OF THE INVENTION

The object of the present invention is to provide Termamyl-like amylases, which variants have alpha-amylase activity and exhibits altered stability at high temperatures and/or at low pH, in particular at low calcium concentrations.

### Termamyl-like alpha-amylases

A number of alpha-amylases produced by Bacillus spp. are highly homologous (identical) on the amino acid level.
The identity of a number of known Bacillus alpha-amylases can be found in the below Table 1:

**Table 1**

| | Percent identity | | | | | | | |
|---|---|---|---|---|---|---|---|---|
| | 707 | AP1378 | BAN | BSG | SP690 | SP722 | AA560 | Termamyl |
| 707 | 100.0 | 86.4 | 66.9 | 66.5 | 87.6 | 86.2 | 95.5 | 68.1 |
| AP1378 | 86.4 | 100.0 | 67.1 | 68.1 | 95.1 | 86.6 | 86.0 | 69.4 |
| BAN | 66.9 | 67.1 | 100.0 | 65.6 | 67.1 | 68.8 | 66.9 | 80.7 |
| BSG | 66.5 | 68.1 | 65.6 | 100.0 | 67.9 | 67.1 | 66.3 | 65.4 |
| SP690 | 87.6 | 95.1 | 67.1 | 67.9 | 100.0 | 87.2 | 87.0 | 69.2 |
| SP722 | 86.2 | 86.6 | 68.8 | 67.1 | 87.2 | 100.0 | 86.8 | 70.8 |
| AA560 | 95.5 | 86.0 | 66.9 | 66.3 | 87.0 | 86.8 | 100.0 | 68.3 |
| Termamyl | 68.1 | 69.4 | 80.7 | 65.4 | 69.2 | 70.8 | 68.3 | 100.0 |

For instance, the B. licheniformis alpha-amylase comprising the amino acid sequence shown in SEQ ID NO: 8 (commercially available as Termamyl^{™}) has been found to be about 81% homologous with the B. amyloliquefaciens alpha-amylase comprising the amino acid sequence shown in SEQ ID NO: 10 and about 65% homologous with the B. stearothermophilus alpha-amylase (BSG) comprising the amino acid sequence shown in SEQ ID NO: 6. Further homologous alpha-amylases include SP690 and SP722 disclosed in WO 95/26397 and further depicted in SEQ ID NO: 2 and SEQ ID NO: 4, respectively, herein. Other amylases are the AA560 alpha-amylase derived from Bacillus sp. and shown in SEQ ID NO: 12, and the #707 alpha-amylase derived from Bacillus sp., shown in SEQ ID NO: 13 and described by Tsukamoto et al., Biochemical and Biophysical Research Communications, 151 (1988), pp. 25-31.

The KSM AP1378 alpha-amylase is disclosed in WO 97/00324 (from KAO Corporation).

Still further homologous alpha-amylases include the alpha-amylase produced by the B. licheniformis strain described in EP 0252666 (ATCC 27811), and the alpha-amylases identified in WO 91/00353 and WO 94/18314. Other commercial Termamyl-like alpha-amylases are comprised in the products sold under the following tradenames: Optitherm^{™} and Takatherm^{™} (Solvay); Maxamyl^{™} (available from Gist-brocades/Genencor), Spezym AA™ and Spezyme Delta AATM (available from Genencor), and Keistase^{™} (available from Daiwa), Dex lo, GC 521 (available from Genencor) and Ultraphlow (from Enzyme Biosystems).

Because of the substantial homology found between these alpha-amylases, they are considered to belong to the same class of alpha-amylases, namely the class of "Termamyl-like alpha-amylases".

Accordingly, in the present context, the term "Termamyl-like" alpha-amylase" is intended to indicate an alpha-amylase, in particular Bacillus alpha-amylase, which, at the amino acid level, exhibits a substantial identity to Termamyl^{™}, i.e., the B. licheniformis alpha-amylase having the amino acid sequence shown in SEQ ID NO: 8, herein.

In other words, all the following alpha-amylases, which has the amino acid sequences shown in SEQ ID NOS: 2, 4, 6, 8, 10, 12 and 13 herein are considered to be "Termamyl-like alpha-amylase". Other Termamyl-like alpha-amylases are alpha-amylases i) which displays at least 60%, such as at least 70%, e.g., at least 75%, or at least 80%, at least 85%, at least 90%, at least 95%, at least 97%, at least 99% homology (identity) with at least one of said amino acid sequences shown in SEQ ID NOS: 2, 4, 6, 8, 10, 12, and 13, and/or is encoded by a DNA sequence which hybridizes to the DNA sequences encoding the above-specified alpha-amylases which are apparent from SEQ ID NOS: 1, 3, 5, 7, 9, and of the present specification (which encoding sequences encode the amino acid sequences shown in SEQ ID NOS: 2, 4, 6, 8, 10 and 12 herein, respectively).

### Homology

The homology may be determined as the degree of identity between the two sequences indicating a derivation of the first sequence from the second. The homology may suitably be determined by means of computer programs known in the art such as GAP provided in the GCG program package (described above). Thus, Gap GCGv8 may be used with the default scoring matrix for identity and the following default parameters: GAP creation penalty of 5.0 and GAP extension penalty of 0.3, respectively for nucleic acidic sequence comparison, and GAP creation penalty of 3.0 and GAP extension penalty of 0.1, respectively, for protein sequence comparison. GAP uses the method of Needleman and Wunsch, (1970), J.Mol. Biol. 48, p.443-453, to make alignments and to calculate the identity.

A structural alignment between Termamyl (SEQ ID NO: 8) and, e.g., another alpha-amylase may be used to identify equivalent/corresponding positions in other Termamyl-like alpha-amylases. One method of obtaining said structural alignment is to use the Pile Up programme from the GCG package using default values of gap penalties, i.e., a gap creation penalty of 3.0 and gap extension penalty of 0.1. Other structural alignment methods include the hydrophobic cluster analysis (Gaboriaud et al., (1987), FEBS LETTERS 224, pp. 149-155) and reverse threading (Huber, T; Torda, AE, PROTEIN SCIENCE Vol. 7, No. 1 pp. 142-149 (1998).

### Hybridisation

The oligonucleotide probe used in the characterisation of the Termamyl-like alpha-amylase above may suitably be prepared on the basis of the full or partial nucleotide or amino acid sequence of the alpha-amylase in question.

Suitable conditions for testing hybridisation involve presoaking in 5xSSC and prehybridizing for 1 hour at 40°C in a solution of 20% formamide, 5xDenhardt's solution, 50mM sodium phosphate, pH 6.8, and 50mg of denatured sonicated calf thymus DNA, followed by hybridisation in the same solution supplemented with 100 mM ATP for 18 hours at 40°C, followed by three times washing of the filter in 2xSSC, 0.2% SDS at 40°C for 30 minutes (low stringency), preferred at 50°C (medium stringency), more preferably at 65°C (high stringency), even more preferably at 75°C (very high stringency). More details about the hybridisation method can be found in Sambrook et al., Molecular Cloning: A Laboratory Manual, 2nd Ed., Cold Spring Harbor, 1989.

In the present context, "derived from" is intended not only to indicate an alpha-amylase produced or producible by a strain of the organism in question, but also an alpha-amylase encoded by a DNA sequence isolated from such strain and produced in a host organism transformed with said DNA sequence. Finally, the term is intended to indicate an alpha-amylase, which is encoded by a DNA sequence of synthetic and/or cDNA origin and which has the identifying characteristics of the alpha-amylase in question. The term is also intended to indicate that the parent alpha-amylase may be a variant of a naturally occurring alpha-amylase, i.e., a variant, which is the result of a modification (insertion, substitution, deletion) of one or more amino acid residues of the naturally occurring alpha-amylase.

### Parent Termamyl-like Alpha-amylases

According to the invention all Termamy-like alpha-amylases, as defined above, may be used as the parent (i.e., backbone) alpha-amylase. In a preferred embodiment of the invention the parent alpha-amylase is derived from B. licheniformis, e.g., one of those referred to above, such as the B. licheniformis alpha-amylase having the amino acid sequence shown in SEQ ID NO: 8.

### Parent hybrid Termamyl-like Alpha-amylases

The parent alpha-amylase (i.e., backbone alpha-amylase) may also be a hybrid alpha-amylase, i.e., an alpha-amylase, which comprises a combination of partial amino acid sequences derived from at least two alpha-amylases.

The parent hybrid alpha-amylase may be one, which on the basis of amino acid homology (identity) and/or DNA hybridization (as defined above) can be determined to belong to the Termamyl-like alpha-amylase family. In this case, the hybrid alpha-amylase is typically composed of at least one part of a Termamyl-like alpha-amylase and part(s) of one or more other alpha-amylases selected from Termamyl-like alpha-amylases or non-Termamyl-like alpha-amylases of microbial (bacterial or fungal) and/or mammalian origin.

Thus, the parent hybrid alpha-amylase may comprise a combination of partial amino acid sequences deriving from at least two Termamyl-like alpha-amylases, or from at least one Termamyl-like and at least one non-Termamyl-like bacterial alpha-amylase, or from at least one Termamyl-like and at least one fungal alpha-amylase. The Termamyl-like alpha-amylase from which a partial amino acid sequence derives, may be any of the specific Termamyl-like alpha-amylase referred to herein.

For instance, the parent alpha-amylase may comprise a C-terminal part of an alpha-amylase derived from a strain of B. licheniformis, and a N-terminal part of an alpha-amylase derived from a strain of B. amyloliquefaciens or from a strain of B. stearothermophilus. For instance, the parent alpha-amylase may comprise at least 430 amino acid residues of the C-terminal part of the B. licheniformis alpha-amylase, and may, e.g., comprise a) an amino acid segment corresponding to the 37 N-terminal amino acid residues of the B. amyloliquefaciens alpha-amylase having the amino acid sequence shown in SEQ ID NO: 10 and an amino acid segment corresponding to the 445 C-terminal amino acid residues of the B. licheniformis alpha-amylase having the amino acid sequence shown in SEQ ID NO: 8, or a hybrid Termamyl-like alpha-amylase being identical to the Termamyl sequence, i.e., the Bacillus licheniformis alpha-amylase shown in SEQ ID NO: 8, except that the N-terminal 35 amino acid residues (of the mature protein) has been replaced by the N-terminal 33 residues of BAN (mature protein), i.e., the Bacillus amyloliquefaciens alpha-amylase shown in SEQ ID NO: 10; or b) an amino acid segment corresponding to the 68 N-terminal amino acid residues of the B. stearothermophilus alpha-amylase having the amino acid sequence shown in SEQ ID NO: 6 and an amino acid segment corresponding to the 415 C-terminal amino acid residues of the B. licheniformis alpha-amylase having the amino acid sequence shown in SEQ ID NO: 8.

Another suitable parent hybrid alpha-amylase is the one previously described in WO 96/23874 (from Novo Nordisk) constituting the N-terminus of BAN, Bacillus amyloliquefaciens alpha-amylase (amino acids 1-300 of the mature protein) and the C-terminus from Termamyl (amino acids 301-483 of the mature protein).

In a preferred embodiment of the invention the parent Termamyl-like alpha-amylase is a hybrid alpha-amylase of SEQ ID NO: 8 and SEQ ID NO: 10. Specifically, the parent hybrid Termamyl-like alpha-amylase may be a hybrid alpha-amylase comprising the 445 C-terminal amino acid residues of the B. licheniformis alpha-amylase shown in SEQ ID NO: 8 and the 37 N-terminal amino acid residues of the alpha-amylase derived from B. amyloliquefaciens shown in SEQ ID NO: 10, which may suitably **further have the following mutations :** H156Y+A181T+N190F+A209V+Q264S (using the numbering in SEQ ID NO: 8). The latter mentioned hybrid is used in the examples below and is referred to as LE174.

Other specifically contemplated parent alpha-amylase include LE174 with fewer mutations, i.e., the right above mentioned hydrid having the following mutations: A181T+N190F+A209V+Q264S; N190F+A209V+Q264S; A209V+Q264S; Q264S; H156Y+N190F+A209V+Q264S; H156Y+A209V+Q264S; H156Y+Q264S; H156Y+A181T+A209V+Q264S; H156Y+A181T+Q264S; H156Y+Q264S; H156Y+A181T+N190F+Q264S; H156Y+A181T+N190F; H156Y+A181T+N190F+A209V. These hybrids are also considered to be part of the invention.

In a preferred embodiment the parent Termamyl-like alpha amylase is LE174, SP722, or AA560 including any of

LE174+G48A+T49I+G107A+I201F; LE174+M197L; LE174+G48A+T49I+G107A+M197L+I201F, or SP722+D183*+G184*;
SP722+D183*+G184*+N195F; SP722+D183*+G184*+M202L;
SP722+D183*+G184*+N195F+M202L; BSG+I181*+G182*;
BSG+I181*+G182*+N193F; BSG+I181*+G182*+M200L;
BSG+I181*+G182*+N193F+M200L;
AA560+D183*+G184*; AA560+D183*+G184*+N195F;
AA560+D183*+G184*+M202L; AA560+D183*+G184*+N195F+M202L.

Other parent alpha-amylases contemplated include LE429, which is LE174 with an additional substitution in I201F. According to the invention LE335 is the alpha-amylase, which in comparison to LE429 has additional substitutions in T49I+G107A; LE399 is LE335+G48A, i.e., LE174, with G48A+T49I+G107A+I201F.

### Altered properties

The following section discusses the relationship between mutations, which are present in variants of the invention, and desirable alterations in properties (relative to those of a parent Termamyl-like alpha-amylase), which may result therefrom.

As mentioned above the invention relates to Termamyl-like alpha-amylases with altered properties (as mentioned above), in particular at high temperatures and/or at low pH, in particular at low calcium concentrations.

In the context of the present invention "high temperature" means temperatures from 70-120°C, preferably 80-100°C, especially 85-95°C.

In the context of the present invention the term "low pH" means from a pH in the range from 4-6, preferably 4.2-5.5, especially 4.5-5.

In the context of the present invention the term "high pH" means from a pH in the range from 8-11, especially 8.5-10.6.

In the context of the present invention the term "low calcium concentration" means free calcium levels lower than 60 ppm, preferably 40 ppm, more preferably 25 ppm, especially 5 ppm calcium.

Parent Termamyl-like alpha-amylase specifically contemplated in connection with going through the specifically contemplated altered properties are the above mentioned parent Termamyl-like alpha-amylase and parent hydrid Termamyl-like alpha-amylases.

The Termamyl® alpha-amylase is used as the starting point, but corresponding positions in, e.g., the SP722, BSG, BAN, AA560, SP690, KSM AP1378, and #707 should be understood as disclosed and specifically comtemplated too.

In a preferred embodiment the variant of the invention has in particular at high temperatures and/or at low pH.

In an aspect the invention relates to variant with altered properties as mentioned above.

In the first aspect a variant of a parent Termamyl-like alpha-amylase, comprising an alteration at one or more positions (using SEQ ID NO: 8 for the amino acid numbering) selected from the group of:
49, 60, 104, 132, 161, 170, 176, 179, 180, 181, 183, 200, 203, 204, 207, 212, 237, 239, 250, 280, 298, 318, 374, 385, 393, 402, 406, 427, 430, 440, 444, 447, 482,
wherein
(a) the alteration(s) are independently
   (i) an insertion of an amino acid downstream of the amino acid which occupies the position,
   (ii) a deletion of the amino acid which occupies the position, or
   (iii) a substitution of the amino acid which occupies the position with a different amino acid,
(b) the variant has alpha-amylase activity and (c) each position corresponds to a position of the amino acid sequence of the parent Termamyl-like alpha-amylase having the amino acid sequence shown in SEQ ID NO: 8.

In Termamyl® (SEQ ID NO: 8) such corresponding positions are: T49; D60; N104; E132; D161; K170; K176; G179; K180; A181; D183; D200; Y203; D204; D207; I212; K237; S239; E250; N280; Q298; L318; Q374; E385; Q393; Y402; H406; L427 D430; V440; N444; E447; Q482.

In SP722 (SEQ ID NO: 4) the corresponding positions are:
T51; D62; N106; D134; D163; Q172; K179; G184; K185; A186; D188; D205; M208; D209; X212; L217, K242, S244, N255, N285, S303, M323; D387, N395; Y404; H408; I429; D432; V442; K446; Q449; K484.

Corresponding positions in other parent alpha-amylases can be found by alignment as described above and shown in the alignment in Fig. 1.

In a preferred embodiment the variant of the invention (using SEQ ID NO: 8 (Termamyl^{™}) for the numbering) has one or more of the following substitutions:
T49I; D60N; N104D; E132A,V,P; D161N; K170Q; K176R; G179N; K180T; A181N; D183N; D200N; X203Y; D204S; D207V,E,L,G; X212I; K237P; S239W; E250G,F; N280S; X298Q; L318M; Q374R; E385V; Q393R; Y402F; H406L,W; L427I D430N; V440A; N444R,K; E447Q,K; Q482K.

In a preferred embodiment the variant of the invention (using SEQ ID NO: 4 (SP722) for the numbering) has one or more of the following substitutions:
T51I; D62N; N106D; D134A,V,P; D163N; X172Q; K179R; G184N; K185T; A186N; D188N; D205N; M208Y; D209S; X212V,E,L,G; L217I, K242P, S244W, N255G,F, N285S, S303Q, X323M; D387V, N395R; Y404F; H408L,W; X429I; D432N; V442A; X446R,K; X449Q,K; X484K, using SEQ ID NO: 4 (SP722) for the numbering.

Preferred double, triple and multi-mutations - using SEQ ID NO: 8 as the basis for the numbering - are selected from the group consisting of:
T49I+D60N; T49I+D60N+E132A; T49I+D60N+E132V;
T49I+D60N+E132V+K170Q; T49I+D60N+E132A+K170Q;
T49I+D60N+E132V+K170Q+K176R; T49I+D60N+E132A+K170Q+K176R;
T49I+D60N+E132V+K170Q+K176R+D207V;
T49I+D60N+E132A+K170Q+K176R+D207V;
T49I+D60N+E132V+K170Q+K176R+D207E;
T49I+D60N+E132A+K170Q+K176R+D207E;
T49I+D60N+E132V+K170Q+K176R+D207V+E250G;
T49I+D60N+E132A+K170Q+K176R+D207V+E250G;
T49I+D60N+E132V+K170Q+K176R+D207E+E250G;
T49I+D60N+E132A+K170Q+K176R+D207E+E250G;
T49I+D60N+E132V+K170Q+K176R+D207E+E250G+N280S;
T49I+D60N+E132A+K170Q+K176R+D207E+E250G+N280S;
T49I+D60N+E132V+K170Q+K176R+D207V+E250G+N280S;
T49I+D60N+E132A+K170Q+K176R+D207V+E250G+N280S;
T49I+D60N+E132V+K170Q+K176R+D207V+E250G+N280S+L318M;
T49I+D60N+E132A+K170Q+K176R+D207V+E250G+N280S+L318M;
T49I+D60N+E132V+K170Q+K176R+D207E+E250G+N280S+L318M; T49I+D60N+E132A+K170Q+K176R+D207E+E250G+N280S+L318M; T49I+D60N+E132V+K170Q+K176R+D207V+E250G+N280S+L318M+Q374R; T49I+D60N+E132A+K170Q+K176R+D207V+E250G+N280S+L318M+Q374R; T49I+D60N+E132V+K170Q+K176R+D207E+E250G+N280S+L318M+Q374R; T49I+D60N+E132A+K170Q+K176R+D207E+E250G+N280S+L318M+Q374R; T49I+D60N+E132V+K170Q+K176R+D207V+E250G+N280S+L318M+Q374R+ E385V; T49I+D60N+E132A+K170Q+K176R+D207V+E250G+N280S+L318M+Q374R+ E385V; T49I+D60N+E132V+K170Q+K176R+D207E+E250G+N280S+L318M+Q374R+ E385V; T49I+D60N+E132A+K170Q+K176R+D207E+E250G+N280S+L318M+Q374R+ E385V; T49I+D60N+E132V+K170Q+K176R+D207V+E250G+N280S+L318M+Q373R+ E385V+Q393R; T49I+D60N+E132A+K170Q+K176R+D207V+E250G+N280S+L318M+Q374R+ E385V+Q393R; T49I+D60N+E132V+K170Q+K176R+D207E+E250G+N280S+L318M+Q374R+ E385V+Q393R; T49I+D60N+E132A+K170Q+K176R+D207E+E250G+N280S+L318M+Q374R+E385V+ Q393R; T49I+D60N+E132V+K170Q+K176R+D207V+E250G+N280S+L318M+Q373R+ E385V+Q393R+Y402F; T49I+D60N+E132A+K170Q+K176R+D207V+E250G+N280S+L318M+Q374R+ E385V+Q393R+Y402F; T49I+D60N+E132V+K170Q+K176R+D207E+E250G+N280S+L318M+Q374R+ E385V+Q393R+Y402F; T49I+D60N+E132A+K170Q+K176R+D207E+E250G+N280S+L318M+Q374R+E385V+ Q393R+Y402F; T49I+D60N+E132V+K170Q+K176R+D207V+E250G+N280S+L318M+Q373R+ E385V+Q393R+Y402F+H406L; T49I+D60N+E132A+K170Q+K176R+D207V+E250G+N280S+L318M+Q374R+ E385V+Q393R+Y402F+H406L; T49I+D60N+E132V+K170Q+K176R+D207E+E250G+N280S+L318M+Q374R+ E385V+Q393R+Y402F+H406L;
T49I+D60N+E132A+K170Q+K176R+D207E+E250G+N280S+L318M+Q374R+E385V+ Q393R+Y402F+H406L;
T49I+D60N+E132V+K170Q+K176R+D207V+E250G+N280S+L318M+Q373R+ E385V+Q393R+Y402F+H406L+L427I;
T49I+D60N+E132A+K170Q+K176R+D207V+E250G+N280S+L318M+Q374R+ E385V+Q393R+Y402F+H406L+L427I;
T49I+D60N+E132V+K170Q+K176R+D207E+E250G+N280S+L318M+Q374R+ E385V+Q393R+Y402F+H406L+L427I;
T49I+D60N+E132A+K170Q+K176R+D207E+E250G+N280S+L318M+Q374R+E385V+ Q393R+Y402F+H406L+L427I;
T49I+D60N+E132V+K170Q+K176R+D207V+E250G+N280S+L318M+Q373R+ E385V+Q393R+Y402F+H406L+L427I+V440A;
T49I+D60N+E132A+K170Q+K176R+D207V+E250G+N280S+L318M+Q374R+ E385V+Q393R+Y402F+H406L+L427I+V440A;
T49I+D60N+E132V+K170Q+K176R+D207E+E250G+N280S+L318M+Q374R+ E385V+Q393R+Y402F+H406L+L427I+V440A;
T49I+D60N+E132A+K170Q+K176R+D207E+E250G+N280S+L318M+Q374R+E385V+ Q393R+Y402F+H406L+L427I+V440A;
D60N+E132A; D60N+E132V; D60N+E132V+K170Q; D60N+E132A+K170Q;
D60N+E132V+K170Q+K176R; T49I+D60N+E132A+K170Q+K176R;
D60N+E132V+K170Q+K176R+D207V; T49I+D60N+E132A+K170Q+K176R+D207V;
D60N+E132V+K170Q+K176R+D207E; T49I+D60N+E132A+K170Q+K176R+D207E;
D60N+E132V+K170Q+K176R+D207V+E250G;
D60N+E132A+K170Q+K176R+D207V+E250G;
D60N+E132V+K170Q+K176R+D207E+E250G;
D60N+E132A+K170Q+K176R+D207E+E250G;
D60N+E132V+K170Q+K176R+D207V+E250G+N280S;
D60N+E132A+K170Q+K176R+D207V+E250G+N280S;
D60N+E132V+K170Q+K176R+D207E+E250G+N280S;
D60N+E132A+K170Q+K176R+D207E+E250G+N280S;
D60N+E132V+K170Q+K176R+D207V+E250G+N280S+L318M;
D60N+E132A+K170Q+K176R+D207V+E250G+N280S+L318M;
D60N+E132V+K170Q+K176R+D207E+E250G+N280S+L318M;
D60N+E132A+K170Q+K176R+D207E+E250G+N280S+L318M;
D60N+E132V+K170Q+K176R+D207V+E250G+N280S+L318M+Q374R;
D60N+E132A+K170Q+K176R+D207V+E250G+N280S+L318M+Q374R;
D60N+E132V+K170Q+K176R+D207E+E250G+N280S+L318M+Q374R;
D60N+E132A+K170Q+K176R+D207E+E250G+N280S+L318M+Q374R;
D60N+E132V+K170Q+K176R+D207E+E250G+N280S+L318M+Q374R+E385V;
D60N+E132A+K170Q+K176R+D207E+E250G+N280S+L318M+Q374R+E385V;
D60N+E132V+K170Q+K176R+D207E+E250G+N280S+L318M+Q374R+E385V;
D60N+E132A+K170Q+K176R+D207E+E250G+N280S+L318M+Q374R+E385V;
D60N+E132V+K170Q+K176R+D207V+E250G+N280S+L318M+Q373R+ E385V+Q393R+Y402F;
D60N+E132A+K170Q+K176R+D207V+E250G+N280S+L318M+Q374R+ E385V+Q393R+Y402F;
D60N+E132V+K170Q+K176R+D207E+E250G+N280S+L318M+Q374R+ E385V+Q393R+Y402F;
D60N+E132A+K170Q+K176R+D207E+E250G+N280S+L318M+Q374R+E385V+ Q393R+Y402F;
D60N+E132V+K170Q+K176R+D207V+E250G+N280S+L318M+Q373R+ E385V+Q393R+Y402F+H406L;
D60N+E132A+K170Q+K176R+D207V+E250G+N280S+L318M+Q374R+ E385V+Q393R+Y402F+H406L;
D60N+E132V+K170Q+K176R+D207E+E250G+N280S+L318M+Q374R+ E385V+Q393R+Y402F+H406L;
D60N+E132A+K170Q+K176R+D207E+E250G+N280S+L318M+Q374R+E385V+ Q393R+Y402F+H406L;
D60N+E132V+K170Q+K176R+D207V+E250G+N280S+L318M+Q373R+ E385V+Q393R+Y402F+H406L+L427I;
D60N+E132A+K170Q+K176R+D207V+E250G+N280S+L318M+Q374R+ E385V+Q393R+Y402F+H406L+L427I;
D60N+E132V+K170Q+K176R+D207E+E250G+N280S+L318M+Q374R+ E385V+Q393R+Y402F+H406L+L427I;
D60N+E132A+K170Q+K176R+D207E+E250G+N280S+L318M+Q374R+E385V+ Q393R+Y402F+H406L+L427I;
D60N+E132V+K170Q+K176R+D207V+E250G+N280S+L318M+Q373R+ E385V+Q393R+Y402F+H406L+L427I+V440A;
D60N+E132A+K170Q+K176R+D207V+E250G+N280S+L318M+Q374R+ E385V+Q393R+Y402F+H406L+L427I+V440A; D60N+E132V+K170Q+K176R+D207E+E250G+N280S+L318M+Q374R+ E385V+Q393R+Y402F+H406L+L427I+V440A;
D60N+E132A+K170Q+K176R+D207E+E250G+N280S+L318M+Q374R+E385V+ Q393R+Y402F+H406L+L427I+V440A;
E132V+K170Q; E132A+K170Q; E132V+K170Q+K176R; E132A+K170Q+K176R;
E132V+K170Q+K176R+D207V; E132A+K170Q+K176R+D207V;
E132V+K170Q+K176R+D207E; E132A+K170Q+K176R+D207E;
E132V+K170Q+K176R+D207V+E250G; E132A+K170Q+K176R+D207V+E250G;
E132V+K170Q+K176R+D207E+E250G; E132A+K170Q+K176R+D207E+E250G;
E132V+K170Q+K176R+D207E+E250G+N280S;
E132A+K170Q+K176R+D207E+E250G+N280S;
E132V+K170Q+K176R+D207V+E250G+N280S;
E132A+K170Q+K176R+D207V+E250G+N280S;
E132V+K170Q+K176R+D207V+E250G+N280S+L318M;
E132A+K170Q+K176R+D207V+E250G+N280S+L318M;
E132V+K170Q+K176R+D207E+E250G+N280S+L318M;
E132A+K170Q+K176R+D207E+E250G+N280S+L318M;
E132V+K170Q+K176R+D207V+E250G+N280S+L318M+Q374R;
E132A+K170Q+K176R+D207V+E250G+N280S+L318M+Q374R;
E132V+K170Q+K176R+D207E+E250G+N280S+L318M+Q374R;
E132A+K170Q+K176R+D207E+E250G+N280S+L318M+Q374R;
E132V+K170Q+K176R+D207V+E250G+N280S+L318M+Q374R+E385V;
E132A+K170Q+K176R+D207V+E250G+N280S+L318M+Q374R+E385V;
E132V+K170Q+K176R+D207E+E250G+N280S+L318M+Q374R+E385V;
E132A+K170Q+K176R+D207E+E250G+N280S+L318M+Q374R+E385V;
E132V+K170Q+K176R+D207V+E250G+N280S+L318M+Q373R+E385V+Q393R;
E132A+K170Q+K176R+D207V+E250G+N280S+L318M+Q374R+E385V+Q393R;
E132V+K170Q+K176R+D207E+E250G+N280S+L318M+Q374R+E385V+Q393R;
E132A+K170Q+K176R+D207E+E250G+N280S+L318M+Q374R+E385V+Q393R;
E132V+K170Q+K176R+D207V+E250G+N280S+L318M+Q373R+ E385V+Q393R+Y402F;
E132A+K170Q+K176R+D207V+E250G+N280S+L318M+Q374R+ E385V+Q393R+Y402F;
E132V+K170Q+K176R+D207E+E250G+N280S+L318M+Q374R+ E385V+Q393R+Y402F;
E132A+K170Q+K176R+D207E+E250G+N280S+L318M+Q374R+E385V+ Q393R+Y402F;
E132V+K170Q+K176R+D207V+E250G+N280S+L318M+Q373R+ E385V+Q393R+Y402F+H406L;
E132A+K170Q+K176R+D207V+E250G+N280S+L318M+Q374R+ E385V+Q393R+Y402F+H406L;
E132V+K170Q+K176R+D207E+E250G+N280S+L318M+Q374R+ E385V+Q393R+Y402F+H406L;
E132A+K170Q+K176R+D207E+E250G+N280S+L318M+Q374R+E385V+ Q393R+Y402F+H406L;
E132V+K170Q+K176R+D207V+E250G+N280S+L318M+Q373R+ E385V+Q393R+Y402F+H406L+L427I;
E132A+K170Q+K176R+D207V+E250G+N280S+L318M+Q374R+ E385V+Q393R+Y402F+H406L+L427I;
E132V+K170Q+K176R+D207E+E250G+N280S+L318M+Q374R+ E385V+Q393R+Y402F+H406L+L427I;
E132A+K170Q+K176R+D207E+E250G+N280S+L318M+Q374R+E385V+ Q393R+Y402F+H406L+L427I;
E132V+K170Q+K176R+D207V+E250G+N280S+L318M+Q373R+ E385V+Q393R+Y402F+H406L+L427I+V440A;
E132A+K170Q+K176R+D207V+E250G+N280S+L318M+Q374R+ E385V+Q393R+Y402F+H406L+L427I+V440A;
E132V+K170Q+K176R+D207E+E250G+N280S+L318M+Q374R+ E385V+Q393R+Y402F+H406L+L427I+V440A;
E132A+K170Q+K176R+D207E+E250G+N280S+L318M+Q374R+E385V+ Q393R+Y402F+H406L+L427I+V440A;
K170Q+K176R; K170Q+K176R+D207V; K170Q+K176R+D207E;
K170Q+K176R+D207V+E250G; K170Q+K176R+D207E+E250G;
K170Q+K176R+D207V+E250G+N280S; K170Q+K176R+D207E+E250G+N280S;
K170Q+K176R+D207E+E250G+N280S+L318M;
K170Q+K176R+D207V+E250G+N280S+L318M;
K170Q+K176R+D207E+E250G+N280S+L318M+Q374R;
K170Q+K176R+D207V+E250G+N280S+L318M+Q374R;
K170Q+K176R+D207E+E250G+N280S+L318M+Q374R+E385V;
K170Q+K176R+D207V+E250G+N280S+L318M+Q374R+E385V;
K170Q+K176R+D207V+E250G+N280S+L318M+Q374R+E385V+Q393R;
K170Q+K176R+D207E+E250G+N280S+L318M+Q374R+E385V+Q393R;
K170Q+K176R+D207V+E250G+N280S+L318M+Q374R+E385V+Q393R+Y402F;
K170Q+K176R+D207E+E250G+N280S+L318M+Q374R+E385V+Q393R+Y402F;
K170Q+K176R+D207V+E250G+N280S+L318M+Q373R+385V+Q393R+Y402F+H406L;
K170Q+K176R+D207E+E250G+N280S+L318M+Q374R+ E385V+Q393R+Y402F+H406L;
K170Q+K176R+D207V+E250G+N280S+L318M+Q373R+ E385V+Q393R+Y402F+H406L+L427I;
K170Q+K176R+D207E+E250G+N280S+L318M+Q374R+ E385V+Q393R+Y402F+H406L+L427I;
K170Q+K176R+D207V+E250G+N280S+L318M+Q373R+ E385V+Q393R+Y402F+H406L+L427I+V440A;
K170Q+K176R+D207E+E250G+N280S+L318M+Q374R+ E385V+Q393R+Y402F+H406L+L427I+V440A;
K176R+D207V; K176R+D207E; K176R+D207V+E250G;
K176R+D207E+E250G;K176R+D207V+E250G+N280S;
K176R+D207E+E250G+N280S;K176R+D207E+E250G+N280S+L318M;
K176R+D207V+E250G+N280S+L318M;
K176R+D207E+E250G+N280S+L318M+Q374R;
K176R+D207V+E250G+N280S+L318M+Q374R;
K176R+D207E+E250G+N280S+L318M+Q374R+E385V;
K176R+D207V+E250G+N280S+L318M+Q374R+E385V;
K176R+D207V+E250G+N280S+L318M+Q374R+E385V+Q393R;
K176R+D207E+E250G+N280S+L318M+Q374R+E385V+Q393R;
K176R+D207V+E250G+N280S+L318M+Q374R+E385V+Q393R+Y402F;
K176R+D207E+E250G+N280S+L318M+Q374R+E385V+Q393R+Y402F;
K176R+D207V+E250G+N280S+L318M+Q374R+E385V+Q393R+Y402F+H406L;
K176R+D207V+E250G+N280S+L318M+Q374R+ E385V+Q393R+Y402F+H406L+L427I;
K176R+D207E+E250G+N280S+L318M+Q374R+ E385V+Q393R+Y402F+H406L+L427I;
K176R+D207V+E250G+N280S+L318M+Q373R+ E385V+Q393R+Y402F+H406L+L427I+V440A;
K176R+D207E+E250G+N280S+L318M+Q374R+ E385V+Q393R+Y402F+H406L+L427I+V440A;
D207V+E250G; D207E+E250G;
D207V+E250G+N280S; D207E+E250G+N280S+L318M;
D207V+E250G+N280S+L318M;D207E+E250G+N280S+L318M+Q374R;
D207V+E250G+N280S+L318M+Q374R;
D207E+E250G+N280S+L318M+Q374R+E385V;
D207V+E250G+N280S+L318M+Q374R+E385V;
D207V+E250G+N280S+L318M+Q374R+E385V+Q393R;
D207E+E250G+N280S+L318M+Q374R+E385V+Q393R;
D207V+E250G+N280S+L318M+Q374R+E385V+Q393R+Y402F;
D207E+E250G+N280S+L318M+Q374R+E385V+Q393R+Y402F;
D207V+E250G+N280S+L318M+Q374R+E385V+Q393R+Y402F+H406L;
D207E+E250G+N280S+L318M+Q374R+E385V+Q393R+Y402F+H406L;
D207V+E250G+N280S+L318M+Q374R+E385V+Q393R+Y402F+H406L+L427I;
D207E+E250G+N280S+L318M+Q374R+E385V+Q393R+Y402F+H406L+L427I;
D207V+E250G+N280S+L318M+Q374R+E385V+Q393R+Y402F+H406L+L427I+V440A;
D207E+E250G+N280S+L318M+Q374R+E385V+Q393R+Y402F+H406L+L427I+ V440A; E250G+N280S; E250G+N280S+L318M; E250G+N280S+L318M+Q374R;
E250G+N280S+L318M+Q374R+E385V;
E250G+N280S+L318M+Q374R+E385V+Q393R;
E250G+N280S+L318M+Q374R+E385V+Q393R+Y402F;
E250G+N280S+L318M+Q374R+E385V+Q393R+Y402F+H406L;
E250G+N280S+L318M+Q374R+E385V+Q393R+Y402F+H406L+L427I;
E250G+N280S+L318M+Q373R+E385V+Q393R+Y402F+H406L+L427I+V440A;
N280S+L318M; N280S+L318M+Q374R; N280S+L318M+Q374R+E385V;
N280S+L318M+Q374R+E385V+Q393R;
N280S+L318M+Q374R+E385V+Q393R+Y402F;
N280S+L318M+Q374R+E385V+Q393R+Y402F+H406L;
N280S+L318M+Q374R+E385V+Q393R+Y402F+H406L+L427I;
N280S+L318M+Q374R+E385V+Q393R+Y402F+H406L+L427I+V440A;
L318M+Q374R; L318M+Q374R+E385V; L318M+Q374R+E385V+Q393R;
L318M+Q374R+E385V+Q393R+Y402F;
L318M+Q374R+E385V+Q393R+Y402F+H406L;
L318M+Q374R+E385V+Q393R+Y402F+H406L+L427I;
L318M+Q374R+E385V+Q393R+Y402F+H406L+L427I+V440A;
Q374R+E385V; Q374R+E385V+Q393R; Q374R+E385V+Q393R+Y402F;
Q374R+E385V+Q393R+Y402F+H406L;
Q374R+E385V+Q393R+Y402F+H406L+L427I;
Q374R+E385V+Q393R+Y402F+H406L+L427I+V440A;
E385V+Q393R; E385V+Q393R+Y402F; E385V+Q393R+Y402F+H406L;
E385V+Q393R+Y402F+H406L+L427I;
E385V+Q393R+Y402F+H406L+L427I+V440A;
Q393R+Y402F; Q393R+Y402F+H406L; Q393R+Y402F+H406L+L427I;
Q393R+Y402F+H406L+L427I+V440A; Y402F+H406L;
Y402F+H406L+L427I; Y402F+H406L+L427I+V440A; H406L+L427I;
H406L+L427I+V440A; L427I+V440A;
N104D+D161N+G179N+K180T+A181N+D183N+D200N+D204S+K237P+S239W+
H406W+D430N+N444K+E447Q+Q482K;
D161N+G179N+K180T+A181N+D183N+D200N+D204S+K237P+S239W+H406W+ D430N+N444K+E447Q+Q482K;
D161N+A181N+D183N+D200N+D204S+K237P+S239W+H406W+ D430N+N444K+E447Q+Q482K;
D161N+A181N+D183N+D200N+D204S+K237P+S239W+H406W+ D430N+E447Q+Q482K;
N104D+D161N+G179N+K180T+A181N+D183N+D200N+D204S+K237P+S239W+ H406W+D430N+E447Q+Q482K;
D161N+G179N+K180T+A181N+D183N+D200N+D204S+K237P+S239W+H406W+ D430N+E447Q+Q482K;
N104D+D161N+G179N+K180T+A181N+D183N+D200N+D204S+K237P+S239W+ H406W+D430N;
D161N+G179N+K180T+A181N+D183N+D200N+D204S+K237P+S239W+H406W+ D430N;
H406W+D430N; N444K+E447Q+Q482K; E447Q+Q482K;
N104D+D161N+G179N+K180T+A181N+D183N+D200N+D204S+K237P+S239W+ H406W+D430N+N444R+N444K+E447K+Q482K;
D161N+G179N+K180T+A181N+D183N+D200N+D204S+K237P+S239W+H406W+ D430N+N444R+N444K+E447K+Q482K;
N104D+D161N+G179N+K180T+A181N+D183N+D200N+D204S+K237P+S239W;
D161N+G179N+K180T+A181N+D183N+D200N+D204S+K237P+S239W;
H406W+D430N; N444K+E447K+Q482K; E447K+Q482K;
N104D+D161N+A181N+D183N+D200N+D204S+K237P+S239W;
N104D+D161N+A181N+D183N+D200N+D204S+K237P;
N104D+D161N+A181N+D183N+D200N+D204S;
D161N+A181N+D183N+D200N+D204S+K237P+S239W;
D161N+A181N+D183N+D200N+D204S+K237P;
D161N+A181N+D183N+D200N+D204S; K237P+S239W, using SEQ ID NO: 8 for the numbering.

In a preferred embodiment the variant has the following substitutions: K170Q+D207V+N280S; E132A+D207V; D207E+E250G+H406L+L427I; D207V+L318M; D60N+D207V+L318M; T49I+E132V+V440A; T49I+K176R+D207V+Y402F; Q374R+E385V+Q393R; N190F+A209V+Q264S; G48A+T49I+G107A+I201F; T49I+G107A+I201F; G48A+T49I+I201F; G48A+T49I+G107A; T49I+I201F; T49I+G107A; G48A+T49I;
D161N+G179N+K180T+A181N+D183N+D200N+D204S+K237P+S239W+H406W+ D430N+N444K+E447Q+Q482K using SEQ ID NO: 8 for the numbering. Specific variant include: LE399; LE174+G48A+T49I+G107A; LE174+G48A+T49I+I201F; LE174+G48A+G107A+I201F; LE174+T49I+G107A+I201F; LE174+G48A+T49I; LE174+G48A; LE174+G107A+I201F; LE174+I201F, are specifically contemplated variants of the invention.

### Stability

In the context of the present invention, mutations (including amino acid substitutionsa and deletion) of importance with respect to achieving altered stability, in particular improved stability (i.e., higher or lower), at especially high temperatures (i.e., 70-120°C) and/or extreme pH (i.e. low or high pH, i.e, pH 4-6 or pH 8-11, respectively), in particular at free (i.e., unbound, therefore in solution) calcium concentrations below 60 ppm, include any of the mutations listed in the "Altered properties" section. The stability may be determined as described in the "Materials & Methods" section below.

### General mutations in variants of the invention

A variant of the invention may in one embodiment comprise one or more modifications in addition to those outlined above. Thus, it may be advantageous that one or more Proline (Pro) residues present in the part of the alpha-amylase variant which is modified is/are replaced with a non-Proline residue which may be any of the possible, naturally occurring non-Proline residues, and which preferably is an Alanine, Glycine, Serine, Threonine, Valine or Leucine.

Analogously, in one embodiment one or more Cysteine residues present in the parent alpha-amylase may be replaced with a non-Cysteine residue such as Serine, Alanine, Threonine, Glycine, Valine or Leucine.

Furthermore, a variant of the invention may - either as the only modification or in combination with any of the above outlined modifications - be modified so that one or more Asp and/or Glu present in an amino acid fragment corresponding to the amino acid fragment 185-209 of SEQ ID NO: 10 is replaced by an Asn and/or Gln, respectively. Also of interest is the replacement, in the Termamyl-like alpha-amylase, of one or more of the Lys residues present in an amino acid fragment corresponding to the amino acid fragment 185-209 of SEQ ID NO: 10 by an Arg.

It is to be understood that the present invention encompasses variants incorporating two or more of the above outlined modifications.

Furthermore, it may be advantageous to introduce mutations in one or more of the following positions (using SEQ ID NO: 8 (Termamyl) for the numbering):
M15, V128, A111, H133, W138, T149, M197, N188, A209, A210, H405, T412, in particular the following single, double or triple or multi mutations:
   M15X, in particular M15T,L;
   V128X, in particular V128E;
   H133X, in particular H133Y;
   N188X, in particular N188S,T,P;
   M197X, in particular M197T,L;
   A209X, in particular A209V;
   M197T/W138F; M197T/W138Y; M15T/H133Y/N188S;
   M15/V128E/H133Y/N188S; E119C/S130C; D124C/R127C; H133Y/T149I; G475R, H133Y/S187D; H133Y/A209V.

### Methods for preparing alpha-amylase variants of the invention

Several methods for introducing mutations into genes are known in the art. After a brief description of cloning of alpha-amylase-encoding DNA sequences, methods for generating mutations at specific sites within the alpha-amylase-encoding sequence will be discribed.

### Cloning a DNA sequence encoding an alpha-amylase

The DNA sequence encoding a parent alpha-amylase may be isolated from any cell or microorganism producing the alpha-amylase in question, using various methods well known in the art. First, a genomic DNA and/or cDNA library should be constructed using chromosomal DNA or messenger RNA from the organism that produces the alpha-amylase to be studied. Then, if the amino acid sequence of the alpha-amylase is known, homologous, labeled oligonucleotide probes may be synthesized and used to identify alpha-amylase-encoding clones from a genomic library prepared from the organism in question. Alternatively, a labeled oligonucleotide probe containing sequences homologous to a known alpha-amylase gene could be used as a probe to identify alpha-amylase-encoding clones, using hybridization and washing conditions of lower stringency.

Yet another method for identifying alpha-amylase-encoding clones would involve inserting fragments of genomic DNA into an expression vector, such as a plasmid, transforming alpha-amylase-negative bacteria with the resulting genomic DNA library, and then plating the transformed bacteria onto agar containing a substrate for alpha-amylase, thereby allowing clones expressing the alpha-amylase to be identified.

Alternatively, the DNA sequence encoding the enzyme may be prepared synthetically by established standard methods, e.g., the phosphoroamidite method described by S.L. Beaucage and M.H. Caruthers, Tetrahedron Letters 22, 1981, pp. 1859-1869, or the method described by Matthes et al., The EMBO J. 3, 1984, pp. 801-805. In the phosphoroamidite method, oligonucleotides are synthesized, e.g., in an automatic DNA synthesizer, purified, annealed, ligated and cloned in appropriate vectors.

Finally, the DNA sequence may be of mixed genomic and synthetic origin, mixed synthetic and cDNA origin or mixed genomic and cDNA origin, prepared by ligating fragments of synthetic, genomic or cDNA origin (as appropriate, the fragments corresponding to various parts of the entire DNA sequence), in accordance with standard techniques. The DNA sequence may also be prepared by polymerase chain reaction (PCR) using specific primers, for instance as described in US 4,683,202 or R.K. Saiki et al., Science 239, 1988, pp. 487-491.

### Site-directed mutagenesis

Once an alpha-amylase-encoding DNA sequence has been isolated, and desirable sites for mutation identified, mutations may be introduced using synthetic oligonucleotides. These oligonucleotides contain nucleotide sequences flanking the desired mutation sites; mutant nucleotides are inserted during oligonucleotide synthesis. In a specific method, a single-stranded gap of DNA, bridging the alpha-amylase-encoding sequence, is created in a vector carrying the alpha-amylase gene. Then the synthetic nucleotide, bearing the desired mutation, is annealed to a homologous portion of the single-stranded DNA. The remaining gap is then filled in with DNA polymerase I (Klenow fragment) and the construct is ligated using T4 ligase. A specific example of this method is described in Morinaga et al. (1984). US 4,760,025 disclose the introduction of oligonucleotides encoding multiple mutations by performing minor alterations of the cassette. However, an even greater variety of mutations can be introduced at any one time by the Morinaga method, because a multitude of oligonucleotides, of various lengths, can be introduced.

Another method for introducing mutations into alpha-amylase-encoding DNA sequences is described in Nelson and Long (1989). It involves the 3-step generation of a PCR fragment containing the desired mutation introduced by using a chemically synthesized DNA strand as one of the primers in the PCR reactions. From the PCR-generated fragment, a DNA fragment carrying the mutation may be isolated by cleavage with restriction endonucleases and reinserted into an expression plasmid.

Alternative methods for providing variants of the invention include gene shuffling, e.g., as described in WO 95/22625 (from Affymax Technologies N.V.) or in WO 96/00343 (from Novo Nordisk A/S), or other corresponding techniques resulting in a hybrid enzyme comprising the mutation(s), e.g., substitution(s) and/or deletion(s), in question. Examples of parent alpha-amylases, which suitably may be used for providing a hybrid with the desired mutations(s) according to the invention include the KSM-K36 and KSM-K38 alpha-amylases disclosed in EP 1,022,334 (hereby incorporated by reference).

### Expression of alpha-amylase variants

According to the invention, a DNA sequence encoding the variant produced by methods described above, or by any alternative methods known in the art, can be expressed, in enzyme form, using an expression vector which typically includes control sequences encoding a promoter, operator, ribosome binding site, translation initiation signal, and, optionally, a repressor gene or various activator genes.

The recombinant expression vector carrying the DNA sequence encoding an alpha-amylase variant of the invention may be any vector, which may conveniently be subjected to recombinant DNA procedures, and the choice of vector will often depend on the host cell into which it is to be introduced. Thus, the vector may be an autonomously replicating vector, i.e., a vector which exists as an extrachromosomal entity, the replication of which is independent of chromosomal replication, e.g., a plasmid, a bacteriophage or an extrachromosomal element, minichromosome or an artificial chromosome. Alternatively, the vector may be one which, when introduced into a host cell, is integrated into the host cell genome and replicated together with the chromosome(s) into which it has been integrated.

In the vector, the DNA sequence should be operably connected to a suitable promoter sequence. The promoter may be any DNA sequence, which shows transcriptional activity in the host cell of choice and may be derived from genes encoding proteins either homologous or heterologous to the host cell. Examples of suitable promoters for directing the transcription of the DNA sequence encoding an alpha-amylase variant of the invention, especially in a bacterial host, are the promoter of the lac operon of E.coli, the Streptomyces coelicolor agarase gene dagA promoters, the promoters of the Bacillus licheniformis alpha-amylase gene (amyL), the promoters of the Bacillus stearothermophilus maltogenic amylase gene (amyM), the promoters of the Bacillus amyloliquefaciens alpha-amylase (amyQ), the promoters of the Bacillus subtilis xylA and xylB genes etc. For transcription in a fungal host, examples of useful promoters are those derived from the gene encoding A. oryzae TAKA amylase, Rhizomucor miehei aspartic proteinase, A. niger neutral alpha-amylase, A. niger acid stable alpha-amylase, A. niger glucoamylase, Rhizomucor miehei lipase, A. oryzae alkaline protease, A. oryzae triose phosphate isomerase or A. nidulans acetamidase.

The expression vector of the invention may also comprise a suitable transcription terminator and, in eukaryotes, polyadenylation sequences operably connected to the DNA sequence encoding the alpha-amylase variant of the invention. Termination and polyadenylation sequences may suitably be derived from the same sources as the promoter.

The vector may further comprise a DNA sequence enabling the vector to replicate in the host cell in question. Examples of such sequences are the origins of replication of plasmids pUC19, pACYC177, pUB110, pE194, pAMB1 and pIJ702.

The vector may also comprise a selectable marker, e.g. a gene the product of which complements a defect in the host cell, such as the dal genes from B. subtilis or B. licheniformis, or one which confers antibiotic resistance such as ampicillin, kanamycin, chloramphenicol or tetracyclin resistance. Furthermore, the vector may comprise Aspergillus selection markers such as amdS, argB, niaD and sC, a marker giving rise to hygromycin resistance, or the selection may be accomplished by co-transformation, e.g., as described in WO 91/17243.

While intracellular expression may be advantageous in some respects, e.g., when using certain bacteria as host cells, it is generally preferred that the expression is extracellular. In general, the Bacillus alpha-amylases mentioned herein comprise a preregion permitting secretion of the expressed protease into the culture medium. If desirable, this preregion may be replaced by a different preregion or signal sequence, conveniently accomplished by substitution of the DNA sequences encoding the respective preregions.

The procedures used to ligate the DNA construct of the invention encoding an alpha-amylase variant, the promoter, terminator and other elements, respectively, and to insert them into suitable vectors containing the information necessary for replication, are well known to persons skilled in the art (cf., for instance, Sambrook et al., Molecular Cloning: A Laboratory Manual, 2nd Ed., Cold Spring Harbor, 1989).

The cell of the invention, either comprising a DNA construct or an expression vector of the invention as defined above, is advantageously used as a host cell in the recombinant production of an alpha-amylase variant of the invention. The cell may be transformed with the DNA construct of the invention encoding the variant, conveniently by integrating the DNA construct (in one or more copies) in the host chromosome. This integration is generally considered to be an advantage as the DNA sequence is more likely to be stably maintained in the cell. Integration of the DNA constructs into the host chromosome may be performed according to conventional methods, e.g., by homologous or heterologous recombination. Alternatively, the cell may be transformed with an expression vector as described above in connection with the different types of host cells.

The cell of the invention may be a cell of a higher organism such as a mammal or an insect, but is preferably a microbial cell, e.g., a bacterial or a fungal (including yeast) cell.

Examples of suitable bacteria are Gram-positive bacteria such as Bacillus subtilis, Bacillus licheniformis, Bacillus lentus, Bacillus brevis, Bacillus stearothermophilus, Bacillus alkalophilus, Bacillus amyloliquefaciens, Bacillus coagulans, Bacillus circulans, Bacillus lautus, Bacillus megaterium, Bacillus thuringiensis, or Streptomyces lividans or Streptomyces murinus, or gramnegative bacteria such as E.coli. The transformation of the bacteria may, for instance, be effected by protoplast transformation or by using competent cells in a manner known per se.

The yeast organism may favorably be selected from a species of Saccharomyces or Schizosaccharomyces, e.g. Saccharomyces cerevisiae. The filamentous fungus may advantageously belong to a species of Aspergillus, e.g., Aspergillus oryzae or Aspergillus niger. Fungal cells may be transformed by a process involving protoplast formation and transformation of the protoplasts followed by regeneration of the cell wall in a manner known per se. A suitable procedure for transformation of Aspergillus host cells is described in EP 238 023.

In a yet further aspect, the present invention relates to a method of producing an alpha-amylase variant of the invention, which method comprises cultivating a host cell as described above under conditions conducive to the production of the variant and recovering the variant from the cells and/or culture medium.

The medium used to cultivate the cells may be any conventional medium suitable for growing the host cell in question and obtaining expression of the alpha-amylase variant of the invention. Suitable media are available from commercial suppliers or may be prepared according to published recipes (e.g., as described in catalogues of the American Type Culture Collection).

The alpha-amylase variant secreted from the host cells may conveniently be recovered from the culture medium by well-known procedures, including separating the cells from the medium by centrifugation or filtration, and precipitating proteinaceous components of the medium by means of a salt such as ammonium sulphate, followed by the use of chromatographic procedures such as ion exchange chromatography, affinity chromatography, or the like.

### Industrial Applications

The alpha-amylase variants of this invention possess valuable properties allowing for a variety of industrial applications. In particular, enzyme variants of the invention are applicable as a component in washing, dishwashing, and hard surface cleaning detergent compositions.

Variant of the invention with altered properties may be used for starch processes, in particular starch conversion, especially liquefaction of starch (see, e.g., US 3,912,590, EP patent publications Nos. 252 730 and 63 909, WO 99/19467, and WO 96/28567 all references hereby incorporated by reference). Also contemplated are compositions for starch conversion purposes, which may beside the variant of the invention also comprise a AMG, pullulanase, and other alpha-amylases.

Further, variants of the invention are also particularly useful in the production of sweeteners and ethanol (see, e.g., US patent no. 5,231,017 hereby incorporated by reference), such as fuel, drinking and industrial ethanol, from starch or whole grains.

A variant of the invention may also be used for textile desizing (see, e.g., WO 95/21247, US patent 4,643,736, EP 119,920 hereby in corporate by reference).

### Detergent compositions

As mentioned above, variants of the invention may suitably be incorporated in detergent compositions. Reference is made, for example, to WO 96/23874 and WO 97/07202 for further details concerning relevant ingredients of detergent compositions (such as laundry or dishwashing detergents), appropriate methods of formulating the variants in such detergent compositions, and for examples of relevant types of detergent compositions.

Detergent compositions comprising a variant of the invention may additionally comprise one or more other enzymes, such as a protease, a lipase, a peroxidase, another amylolytic enzyme, glucoamylase, maltogenic amylase, CGTase and/or a cellulase, mannanase (such as Mannaway^{™} from Novozymes, Denmark)), pectinase, pectine lyase, cutinase, laccase, and/or another alpha-amylase.

Alpha-amylase variants of the invention may be incorporated in detergents at conventionally employed concentrations. It is at present contemplated that a variant of the invention may be incorporated in an amount corresponding to 0.00001-10 mg (calculated as pure, active enzyme protein) of alpha-amylase per liter of wash/dishwash liquor using conventional dosing levels of detergent.

### Compositions

The invention also related to composition comprising a variant of the invention, and in a preferred embodiment also a B. stearothermophilus alpha-amylase (BSG), in particular a variant thereof.

In another embodient the composition comprises beside a variant of the invention a glucoamylase, in particular a glucoamylase originating from Aspergillus niger (e.g., the G1 or G2 A. niger AMG disclosed in Boel et al. (1984), "Glucoamylases G1 and G2 from Aspergillus niger are synthesized from two different but closely related mRNAs", EMBO J. 3 (5), p. 1097-1102, or a variant therefore, in particular a variant disclosed in WO 00/04136 or WO 01/04273 or the Talaromyces emersonii AMG disclosed in WO 99/28448.

A specific combination is LE399 and a variant disclosed in WO 00/04136 or Wo 01/04273, in particular a variant with oe or more of the following substitutions:
N9A,S56A,V59A,S119P,A246T,N313G,E342T,A393R,S394R,Y402F,E408R,
in particular a variant with all mutation.

In an embodiment the composition of the invention also comprises a pullulanase, in particular a Bacillus pullulanase.

### MATERIALS AND METHODS

### Enzymes:

Bacillus licheniformis alpha-amylase shown in SEQ ID NO: 8 and also available from Novozymes.
AA560: SEQ ID NO: 12; disclosed in WO 00/60060; deposited on 25th January 1999 at DSMZ and assigned the DSMZ no. 12649.
AA560 were deposited by the inventors under the terms of the Budapest Treaty on the International Recognition of the Deposit of Microorganisms for the Purposes of Patent Procedure at Deutshe Sammmlung von Microorganismen und Zellkulturen GmbH (DSMZ), Mascheroder Weg 1b, D-38124 Braunschweig DE.

LB medium (In 1 liter H2O: 10 g bacto-tryptone, 5 g bacto-yeast extract, 10 g NaCl, pH adjusted to 7.0 w. NaOH, autoclaved).

TY agar plates (In 1 liter H2O: 16 g bacto-tryptone, 10 g bacto-yeast extract, 5 g NaCl, pH adjusted to 7.0 w. NaOH, and 15 g bacto-agar is added prior to autoclaving).

10% Lugol solution (Iodine/Potassium iodine solution; made by 10-fold dil. in H2O of stock: Sigma Cat. no. L 6146).

Bacillus subtilis SHA273: see WO 95/10603

### Plasmids

pDN1528 contains the complete gene encoding Termamyl, amyL, the expression of which is directed by its own promoter. Further, the plasmid contains the origin of replication, ori, from plasmid pUB110 and the cat gene from plasmid pC194 conferring resistance towards chloramphenicol. pDN1528 is shown in Fig. 9 of WO 96/23874.

### Methods:

### Low pH filter assay

Bacillus libraries are plated on a sandwich of cellulose acetate (OE 67, Schleicher & Schuell, Dassel, Germany) - and nitrocellulose filters (Protran-Ba 85, Schleicher & Schuell, Dassel, Germany) on TY agar plates with 10 micro g/ml chloramphenicol at 37°C for at least 21 hours. The cellulose acetate layer is located on the TY agar plate.

Each filter sandwich is specifically marked with a needle after plating, but before incubation in order to be able to localize positive variants on the filter, and the nitrocellulose filter with bound variants is transferred to a container with citrate buffer, pH 4.5 and incubated at 80°C for 20 minutes (when screening for variants in the wild type backbone) or 85oC for 60 minutes (when screening for variants in the LE399 backbone). The cellulose acetate filters with colonies are stored on the TY-plates at room temperature until use. After incubation, residual activity is detected on assay plates containing 1% agarose, 0.2% starch in citrate buffer, pH 6.0. The assay plates with nitrocellulose filters are marked the same way as the filter sandwich and incubated for 2 hours at 50°C. After removal of the filters the assay plates are stained with 10% Lugol solution. Starch degrading variants are detected as white spots on dark blue background and then identified on the storage plates. Positive variants are re-screened twice under the same conditions as the first screen.

### Secondary screening

Positive transformants after rescreening are picked from the storage plate and tested in a secondary plate assay. Positive transformants are grown for 22 hours at 37°C in 5 ml LB + chloramphenicol. The Bacillus culture of each positive transformant and as a control a clone expressing the corresponding backbone are incubated in citrate buffer, pH 4.5 at 90°C and samples are taken at 0, 10, 20, 30, 40, 60 and 80 minutes. A 3 micro liter sample is spotted on an assay plate. The assay plate is stained with 10% Lugol solution. Improved variants are seen as variants with higher residual activity (detected as halos on the assay plate) than the backbone. The improved variants are determined by nucleotide sequencing.
Stability assay of unpurified variants:
Bacillus cultures expressing the variants to be analysed are grown for 21 hours at 37°C in 10 ml LB+chloramphenicol. 800 micro liter culture is mixed with 200 micro 1 citrate buffer, pH 4.5. A number of 70 micro 1 aliquots corresponding to the number of sample time points are made in PCR tubes and incubated at 70°C (for variants in the wt backbone) or 90°C (for variants in LE399) for various time points (typically 5, 10, 15, 20, 25 and 30 minutes) in a PCR machine. The 0 min sample is not incubated at high temperature. Activity in the sample is measured by transferring 20 micro 1 to 200 micro 1 of the alpha-amylase PNP-G7 substrate MPR3 ((Boehringer Mannheim Cat. no. 1660730) as described below under "Assays for Alpha-Amylase Activity". Results are plotted as percentage activity (relative to the 0 time point) versus time, or stated as percentage residual activity after incubation for a certain period of time.

### Fermentation and purification of alpha-amylase variants

A B. subtilis strain harbouring the relevant expression plasmid is streaked on a LB-agar plate with 10 micro g/ml kanamycin from -80°C stock, and grown overnight at 37°C.
The colonies are transferred to 100 ml PS-1 media supplemented with 10 micro g/ml chloamphinicol in a 500 ml shaking flask.

**Composition of PS-1 medium:**

| | |
|---|---|
| Pearl sugar | 100 g/l |
| Soy Bean Meal | 40 g/l |
| Na2HPO4, 12 H2O | 10 g/l |
| PluronicTM PE 6100 | 0.1 g/l |
| CaCO3 | 5 g/l |

The culture is shaken at 37°C at 270 rpm for 5 days.

Cells and cell debris are removed from the fermentation broth by centrifugation at 4500 rpm in 20-25 minutes. Afterwards the supernatant is filtered to obtain a completely clear solution. The filtrate is concentrated and washed on a UF-filter (10000 cut off membrane) and the buffer is changed to 20mM Acetate pH 5.5. The UF-filtrate is applied on a S-sepharose F.F. and elution is carried out by step elution with 0.2M NaCl in the same buffer. The eluate is dialysed against 10mM Tris, pH 9.0 and applied on a Q-sepharose F.F. and eluted with a linear gradient from 0-0.3M NaCl over 6 column volumes. The fractions that contain the activity (measured by the Phadebas assay) are pooled, pH was adjusted to pH 7.5 and remaining color was removed by a treatment with 0.5% W/vol. active coal in 5 minutes.

### Stability determination of purified variants

All stability trials of purified variants are made using the same set up. The method is as follows:
The enzyme is incubated under the relevant conditions (1-4). Samples are taken at various time points, e.g., after 0, 5, 10, 15 and 30 minutes and diluted 25 times (same dilution for all taken samples) in assay buffer (0.1M 50mM Britton buffer pH 7.3) and the activity is measured using the Phadebas assay (Pharmacia) under standard conditions pH 7.3, 37°C.

The activity measured before incubation (0 minutes) is used as reference (100%). The decline in percent is calculated as a function of the incubation time. The table shows the residual activity after, e.g., 30 minutes of incubation.

### Specific activity determination

The specific activity is determined using the Phadebas assay (Pharmacia) as activity/mg enzyme. The manufactures instructions are followed (see also below under "Assay for α-amylase activity).

### Assays for Alpha-Amylase Activity

### 1. Phadebas assay

Alpha-amylase activity is determined by a method employing Phadebas® tablets as substrate. Phadebas tablets (Phadebas® Amylase Test, supplied by Pharmacia Diagnostic) contain a crosslinked insoluble blue-colored starch polymer, which has been mixed with bovine serum albumin and a buffer substance and tabletted.

For every single measurement one tablet is suspended in a tube containing 5 ml 50 mM Britton-Robinson buffer (50 mM acetic acid, 50 mM phosphoric acid, 50 mM boric acid, 0.1 mM CaCl2, pH adjusted to the value of interest with NaOH). The test is performed in a water bath at the temperature of interest. The alpha-amylase to be tested is diluted in x ml of 50 mM Britton-Robinson buffer. 1 ml of this alpha-amylase solution is added to the 5 ml 50 mM Britton-Robinson buffer. The starch is hydrolyzed by the alpha-amylase giving soluble blue fragments. The absorbance of the resulting blue solution, measured spectrophotometrically at 620 nm, is a function of the alpha-amylase activity.

It is important that the measured 620 nm absorbance after 10 or 15 minutes of incubation (testing time) is in the range of 0.2 to 2.0 absorbance units at 620 nm. In this absorbance range there is linearity between activity and absorbance (Lambert-Beer law). The dilution of the enzyme must therefore be adjusted to fit this criterion. Under a specified set of conditions (temp., pH, reaction time, buffer conditions) 1 mg of a given alpha-amylase will hydrolyze a certain amount of substrate and a blue colour will be produced. The colour intensity is measured at 620 nm. The measured absorbance is directly proportional to the specific activity (activity/mg of pure alpha-amylase protein) of the alpha-amylase in question under the given set of conditions.

### 2. Alternative method

Alpha-amylase activity is determined by a method employing the PNP-G7 substrate. PNP-G7 which is a abbreviation for p-nitrophenyl-alpha,D-maltoheptaoside is a blocked oligosaccharide which can be cleaved by an endo-amylase. Following the cleavage, the alpha-Glucosidase included in the kit digest the substrate to liberate a free PNP molecule which has a yellow colour and thus can be measured by visible spectophometry at λ=405nm (400-420 nm). Kits containing PNP-G7 substrate and alpha-Glucosidase is manufactured by Boehringer-Mannheim (cat. No.1054635).

To prepare the reagent solution 10 ml of substrate/buffer solution is added to 50 ml enzyme/buffer solution as recommended by the manufacturer. The assay is performed by transferring 20 micro l sample to a 96 well microtitre plate and incubating at 25°C. 200 micro l reagent solution pre-equilibrated to 25°C is added. The solution is mixed and pre-incubated 1 minute and absorption is measured every 30 sec. over 4 minutes at OD 405 nm in an ELISA reader.

The slope of the time dependent absorption-curve is directly proportional to the activity of the alpha-amylase in question under the given set of conditions.

### EXAMPLES

### Example 1.

Construction, by error-prone PCR mutagenesis, of Bacillus licheniformis alpha-amylase variants having an improved stability at low pH, high temperature and low calcium ion concentration compared to the parent enzyme.

### Error-prone PCR mutagenesis and library construction

To improve the stability at low pH and low calcium concentration of the parent Bacillus licheniformis alpha-amylase, error-prone PCR mutagenesis was performed. The plasmid pDN1528 encoding the wild-type Bacillus licheniformis alpha-amylase gene was utilized as template to amplify this gene with primers: 22149: 5'-CGA TTG CTG ACG CTG TTA TTT GCG-3' (SEQID NO: 14) and 24814: 5'-GAT CAC CCG CGA TAC CGT C-3' (SEQ ID NO: 15) under PCR conditions where increased error rates leads to introduction of random point mutations. The PCR conditions utilized were: 10 mM Tris-HCl, pH 8.3, 50 mM KCl, 4 mM MgCl2, 0.3 mM MnCl2, 0.1mM dGTP/dATP, 0.5 mM dTTP/dCTP, and 2.5 units Taq polymerase per 100 micro l reaction.

The resultant PCR fragment was purified on gel and used in a PCR-based multimerization step with a gel purified vector fragment created by PCR amplification of pDN1528 with primers #24: 5'-GAA TGT ATG TCG GCC GGC AAA ACG CCG GTG A-3' (SEQ ID NO: 16) and #27: 5'-GCC GCC GCT GCT GCA GAA TGA GGC AGC AAG-3' (SEQ ID NO:17) forming an overlap to the insert fragment. The multimerization reaction was subsequently introduced into B. subtilis (Shafikhani et al., Biotechniques, 23 (1997), 304-310).

### Screening

The error-prone library described above was screened in the low pH filter assay (see "Materials & Methods"). Clones testing positive upon rescreening was submitted to secondary screening for stability in the liquid assay described in Materials and Methods.

### Results:

**Increased stability at pH 4.5, 5 ppm calcium incubated at 90°C**

| | | | | | |
|---|---|---|---|---|---|
| **Name** | wt | LE488 | LE489 | 7.19.1 | 8.9.1 |
| **Mutations** | - | D207V | K170Q | E132A | D207E |
| | | | D207V | D207V | E250G |
| | | | N280S | | H406L |
| | | | | | L427I |
| **Stability1)** | - | + | + | + | + |

| | | | | | |
|---|---|---|---|---|---|
| 1) A "+" indicates significant increase in stability relative to wild type. | | | | | |

**Increased stability at pH 4.5, 5 ppm calcium incubated at 90°C**

| | | | | | | |
|---|---|---|---|---|---|---|
| **Name** | wt | LE491 | LE492 | LE493 | LE494 | 19.3.1 |
| **Mutations** | - | D60N | T49I | T49I | Q374R | N190F |
| | | D207V | E132V | K176R | E385V | A209V |
| | | L318M | V440A | D207V | Q393R | Q264S |
| | | | | Y402F | | |
| **Stability1)** | - | + | + | + | + | + |

| | | | | | | |
|---|---|---|---|---|---|---|
| 1) A "+" indicates significant increase in stability relative to wt. | | | | | | |

**Increased stability at pH 4.5, 5 ppm calcium incubated at 90°C**

| | | | | | |
|---|---|---|---|---|---|
| **Name** | wt | E132-1 | D207-7 | D207-6 | E250-8 |
| **Mutations** | - | E132P | D207L | D207G | E250F |
| **Stability1)** | - | + | + | + | + |

| | | | | | |
|---|---|---|---|---|---|
| 1) A "+" indicates significant increase in stability relative to wt. | | | | | |

### Example 2

Transfer, by site-directed mutagenesis, of a selection of mutations from Example 1 to a new (non-wild type) backbone to improve stability at low pH and low calcium ion concentration compared to the parent enzyme.

### Site-directed mutagenesis

Mutations from LE493 (K176R+D207V+Y402F) were transferred to LE399 yielding LE495. This was performed by the overlap PCR method (Kirchhoff and Desrosiers, PCR Methods and Applications, 2 (1993), 301-304). 2 overlapping PCR fragments were generated by amplification of the LE399 template with the primers: Fragment A: #312 Mut176 5'-CCC GAA AGC TGA ACC GCA TCT ATA GGT TTC AAG GGA AGA CTT GGG ATT-3' (SEQ ID NO: 18) (mutated codon indicated in bold) and #290 D207overlap 5'-AGG ATG GTC ATA ATC AAA GTC GG-3'(SEQ ID NO: 19); Fragment B: #313 Mut207 5'-CCG ACT TTG ATT ATG ACC ATC CTG TTG TCG TAG CAG AGA TTA AGA GAT GGG G-3' (SEQ ID NO: 20) and #314 Mut402 5'-CGA CAA TGT CAT GGT GGT CGA AAA AAT CAT GCT GTG CTC CGT ACG-3' (SEQ ID NO: 21). Fragments A and B were mixed in equimolar ratios and subsequently the full-length fragment was amplified with the external primers: #312 Mut176 and #314 Mut402. This fragment was used in a multimerization reaction with the vector PCR fragment created with the primers #296 Y402multi 5'-TTT CGA CCA CCA TGA CAT TGT CG-3' (SEQ ID NO: 22) and #305 399Multi176 5'-TAT AGA TGC GGT TCA GCT TTC GGG-3' (SEQ ID NO: 23) on template LE399 as described above. The multimerization reaction was subsequently transformed into B. subtilis. Clones were screened for stability in the assay mentioned above. The presence of the mutations from LE493 in several clones with increased stability was confirmed by sequencing.

LE 497 was obtained in a similar manner by amplifying the LE399 encoding template with primers #312 Mut176 and #314 Mut402 and using the resulting PCR fragment in a multimerization reaction with a vector fragment obtained by PCR amplification of the LE399 template with the primers #296 Y402multi and #305 399Multi176.

### Results:

Stabilization of LE399 variant at pH 4.5, 5ppm calcium incubated at 90oC

| | | | |
|---|---|---|---|
| Name | LE399 | LE495 | LE497 |
| Mutations | - | K176R | K176R |
| | (backbone) | D207V | Y402F |
| | | Y402F | |
| Stability1) | - | + | + |

| | | | |
|---|---|---|---|
| 1) A "+" indicates significant increase in stability relative to backbone. | | | |

### PREFERRED EMBODIMENTS

1. A variant of a parent Termamyl-like alpha-amylase, comprising an alteration at one or more positions selected from the group of:
   49, 60, 104, 132, 161, 170, 176, 179, 180, 181, 183, 200, 203, 204, 207, 212, 237, 239, 250, 280, 298, 318, 374, 385, 393, 402, 406, 427, 430, 440, 444, 447, 482,
   wherein
   (a) the alteration(s) are independently
      (i) an insertion of an amino acid downstream of the amino acid which occupies the position,
      (ii) a deletion of the amino acid which occupies the position, or
      (iii) a substitution of the amino acid which occupies the position with a different amino acid,
   (b) the variant has alpha-amylase activity and (c) each position corresponds to a position of the amino acid sequence of the parent Termamyl-like alpha-amylase having the amino acid sequence shown in SEQ ID NO: 8.
2. The variant of embodiment 1, which variant has one or more of the following mutations: T49I; D60N; N104D; E132A,V,P; D161N; K170Q; K176R; G179N; K180T; A181N; D183N; D200N; X203Y; D204S; D207V,E,L,G; X212I; K237P; S239W; E250G,F; N280S; X298Q; L318M; Q374R; E385V; Q393R; Y402F; H406L,W; L427I D430N; V440A; N444R,K; E447Q,K; Q482K using SEQ ID NO: 8 for the numbering.
3. The variant of embodiment 1 or 2, wherein the variant has the following mutations: K170Q+D207V+N280S; E132A+D207V; D207E+E250G+H406L+L427I; D207V+L318M; D60N+D207V+L318M; T49I+E132V+V440A; T49I+K176R+D207V+Y402F; Q374R+E385V+Q393R; N190F+A209V+Q264S; G48A+T49I+G107A+I201F; T49I+G107A+I201F; G48A+T49I+I201F; G48A+T49I+G107A; T49I+I201F; T49I+G107A; G48A+T49I;
   N104D+D161N+G179N+K180T+A181N+D183N+D200N+D204S+K237P+S239W+ H406W+D430N+N444K+E447Q+Q482K;
   D161N+G179N+K180T+A181N+D183N+D200N+D204S+K237P+S239W+H406W+ D430N+N444K+E447Q+Q482K;
   D161N+A181N+D183N+D200N+D204S+K237P+S239W+H406W+ D430N+N444K+E447Q+Q482K;
   D161N+A181N+D183N+D200N+D204S+K237P+S239W+H406W+ D430N+E447Q+Q482K;
   N104D+D161N+G179N+K180T+A181N+D183N+D200N+D204S+K237P+S239W+ H406W+D430N+E447Q+Q482K;
   D161N+G179N+K180T+A181N+D183N+D200N+D204S+K237P+S239W+H406W+ D430N+E447Q+Q482K;
   N104D+D161N+G179N+K180T+A181N+D183N+D200N+D204S+K237P+S239W+ H406W+D430N;
   D161N+G179N+K180T+A181N+D183N+D200N+D204S+K237P+S239W+H406W+ D430N;
   H406W+D430N; N444K+E447Q+Q482K; E447Q+Q482K;
   N104D+D161N+G179N+K180T+A181N+D183N+D200N+D204S+K237P+S239W+ H406W+D430N+N444R+N444K+E447K+Q482K;
   D161N+G179N+K180T+A181N+D183N+D200N+D204S+K237P+S239W+H406W+ D430N+N444R+N444K+E447K+Q482K;
   N104D+D161N+G179N+K180T+A181N+D183N+D200N+D204S+K237P+S239W;
   D161N+G179N+K180T+A181N+D183N+D200N+D204S+K237P+S239W;
   H406W+D430N; N444K+E447K+Q482K; E447K+Q482K;
   N104D+D161N+A181N+D183N+D200N+D204S+K237P+S239W;
   N104D+D161N+A181N+D183N+D200N+D204S+K237P;
   N104D+D161N+A181N+D183N+D200N+D204S;
   D161N+A181N+D183N+D200N+D204S+K237P+S239W;
   D161N+A181N+D183N+D200N+D204S+K237P;
   D161N+A181N+D183N+D200N+D204S; K237P+S239W, using SEQ ID NO: 8 for the numbering.
4. The variant of any of embodiments 1-3, wherein the parent Termamyl-like alpha-amylase is derived from a strain of B. licheniformis (SEQ ID NO: 8), B. amyloliquefaciens (SEQ ID NO: 10), B. stearothermophilus (SEQ ID NO: 6).
5. The variant of any of embodiments 1-4, wherein the parent Termamyl-like amylase is any of:
   LE174; LE174+G48A+T49I+G107A+I201F; LE174+M197L;
   LE174+G48A+T49I+G107A+M197L+I201F.
6. The variant of embodiment 1, wherein the variant is mutated in one or more of the following positions: T51I; D62N; N106D; D134A,V,P; D163N; X172Q; K179R; G184N; K185T; A186N; D188N; D205N; M208Y; D209S; X212V,E,L,G; L217I, K242P, S244W, N255G,F, N285S, S303Q, X323M; D387V, N395R; Y404F; H408L,W; X429I; D432N; V442A; X446R,K; X449Q,K; X484K, using SEQ ID NO: 4 (SP722) for the numbering.
7. The variant of embodiment 1 or 6, wherein the variant has the following mutations: E212V+N285S; D134A+E212V; N255G+H408L+X429I; E212V+X323M; D62N+E212V+X323M; T51I+D134V+V442A; T51I+K179R+E212V+Y404F; D387V+N395R; N195F+X212V+K269S, when using SEQ ID NO: 4 (SP722) for the numbering.
8. The variant of any of embodiments 1-7, wherein the parent Termamyl-like alpha-amylase is selected from the group comprising: SP690 (SEQ ID NO: 2); SP722 (SEQ ID NO: 4; AA560 (SEQ ID NO: 12); #707 alpha-amylase (SEQ ID NO: 13); KSM-AP1378.
9. The variant of any of embodiments 1-8, wherein the parent Termamyl-like amylase is any of: SP722+D183*+G184*;
   SP722+D183*+G184*+N195F; SP722+D183*+G184*+M202L;
   SP722+D183*+G184*+N195F+M202L; BSG+I181*+G182*;
   BSG+I181*+G182*+N193F; BSG+I181*+G182*+M200L;
   BSG+I181*+G182*+N193F+M200L;
   AA560+D183*+G184*; AA560+D183*+G184*+N195F;
   AA560+D183*+G184*+M202L; AA560+D183*+G184*+N195F+M202L.
10. The variant of any of embodiments 1-9, wherein the parent Termamyl-like alpha-amylase has an amino acid sequence which has a degree of identity to SEQ ID NO: 8 of at least 60%, preferably 70%, more preferably at least 80%, even more preferably at least about 90%, even more preferably at least 95%, even more preferably at least 97%, and even more preferably at least 99%.
11. The variant of any of embodiments 1-10, wherein the parent Termamyl-like alpha-amylase is encoded by a nucleic acid sequence, which hydridizes under low, preferably medium, preferred high stringency conditions, with the nucleic acid sequence of SEQ ID NO: 7.
12. The variant of any of embodiments 1-11, which variant has altered stability, in particular at high temperatures from 70-120°C and/or low pH in the range from pH 4-6
13. A DNA construct comprising a DNA sequence encoding an alpha-amylase variant according to any one of embodiments 1-12.
14. A recombinant expression vector which carries a DNA construct according to embodiment 13.
15. A cell which is transformed with a DNA construct according to embodiment 13 or a vector according to embodiment 14.
16. The cell according to embodiment 15, which is a microorganism, preferably a bacterium or a fungus.
17. The cell according to embodiment 16, which cell is a gram-positive bacterium, such as Bacillus subtilis, Bacillus licheniformis, Bacillus lentus, Bacillus brevis, Bacillus stearothermophilus, Bacillus alkalophilus, Bacillus amyloliquefaciens, Bacillus coagulans, Bacillus circulans, Bacillus lautus or Bacillus thuringiensis.
18. A composition comprising an alpha-amylase variant of any of embodiments 1-12.
19. The composition of embodiment 18, further comprising a B. stearothermophilus (BSG) alpha-amylase, in particular SP961, particular in a ratio of 1:10 to 10:1, preferably 1:2.
20. The composition of embodiment 18 or 19, wherein the composition further comprises a glucoamylase, pullulanase and/or a phytase.
21. A detergent composition comprising an alpha-amylase variant according to any of embodiments 1-12.
22. A detergent composition of embodiment 21, which additionally comprises another enzyme such as a protease, a lipase, a peroxidase, another amylolytic enzyme, glucoamylase, maltogenic amylase, CGTase, mannanase, cutinase, laccase and/or a cellulase.
23. Use of an alpha-amylase variant according to any of embodiments 1-12 or a composition according to any of embodiments 18-20 for starch liquefaction.
24. Use of an alpha-amylase variant according to any of embodiments 1-12 or a composition according to embodiment 18-20 for ethanol production.
25. Use of an alpha-amylase variant according to any one of embodiments 1-12 or a composition according to embodiments 18-20 for washing and/or dishwashing.
26. Use of an alpha-amylase variant of any one of embodiments 1-12 or a composition according to embodiment 18-20 for textile desizing.

## Claims

1. A variant of a parent alpha-amylase, which comprises an alteration, wherein
(a) the alteration is a substitution of the amino acid at a position corresponding to position S239 in SEQ ID NO: 8 with a different amino acid;
(b) the variant has alpha-amylase activity;
(c) the parent alpha-amylase displays at least 60% identity with SEQ ID NO: 6 or 8.

2. The variant of claim 1, wherein the variant comprises the substitution S239W.

3. The variant of claim 1 or 2, wherein the parent alpha-amylase displays at least 70%, preferably at least 80%, more preferably at least 90%, more preferred at least 95% identity with SEQ ID NO: 8.

4. The variant of claim 1 or 2, wherein the parent alpha-amylase displays at least 70%, preferably at least 80%, more preferably at least 90%, more preferred at least 95% identity with SEQ ID NO: 6.

5. The variant of any of claims 1-4, wherein the parent alpha-amylase is derived from a strain of *Bacillus.*

6. The variant of any of claims 1-5, wherein the parent alpha-amylase is a strain of *Bacillus licheniformis.*

7. The variant of any of claims 1-5, wherein the parent alpha-amylase is a strain of *Bacillus stearothermophilus.*

8. A DNA construct comprising a DNA sequence encoding an alpha-amylase variant according to any of claims 1-7.

9. A recombinant expression vector which carries a DNA construct according to claim 8.

10. A cell which is transformed with a DNA construct according to claim 8 or a vector according to claim 9.

11. The cell according to claim 10, which is a microorganism, preferably a bacterium or a fungus.

12. The cell according to claim 11, which cell is a gram-positive bacterium, preferably selected from the group of *Bacillus subtilis, Bacillus licheniformis, Bacillus lentus, Bacillus brevis, Bacillus stearothermophilus, Bacillus alkalophilus, Bacillus amyloliquefaciens, Bacillus coagulans, Bacillus circulans, Bacillus lautus* and *Bacillus thuringiensis.*

13. A composition comprising an alpha-amylase variant of any of claims 1-7.

14. The composition of claim 13, wherein the composition further comprises a glucoamylase, pullulanase and/or a phytase.

15. A detergent composition comprising an alpha-amylase variant according to any of claims 1-7.

16. A detergent composition of claim 15, which additionally comprises another enzyme such as a protease, a lipase, a peroxidase, another amylolytic enzyme, glucoamylase, maltogenic amylase, CGTase, mannanase, cutinase, laccase and/or a cellulase.

17. Use of an alpha-amylase variant according to any of claims 1-7 or a composition according to claim 13 or 14 for starch liquefaction.

18. Use of an alpha-amylase variant according to any of claims 1-7 or a composition according to claim 13 or 14 for ethanol production.

19. Use of an alpha-amylase variant according to any of claims 1-7 or a composition according to claim 15 or 16 for washing and/or dishwashing.

20. Use of an alpha-amylase variant according to any of claims 1-7 or a composition according to claim 15 or 16 for textile desizing.
